# EUROPEAN PATENT APPLICATION

(11) **EP 2 343 311 A2**
(43) Date of publication of application: **13.07.2011**
(21) Application number: 10185606.0
(22) Date of filing: 05.09.2005
(51) Int. Cl.: C07K 7/06, C07K 7/08, A61K 38/08, A61K 38/10, A61K 48/00, G01N 33/68, G01N 33/574

(54) **Peptide inhibitors of HK2 and their use**

(30) Priority: 03.09.2004 US 606916 P; 14.07.2005 FI 20050753
(62) Divisional of application: 05779484.4
(71) Applicant: Licentia Oy, 00290 Helsinki (FI)
(72) Inventor: Stenman, Ulf-Håkan, 02700 Kauniainen (FI); Leinonen, Jari, 00610 Helsinki (FI); Närvänen, Ale, 70340 Kuopio (FI)
(74) Representative: Arvela, Sakari Mikael

(57) **Abstract**

Isolated peptide comprising an amino acid sequence selected from SRFKVWWAAG (SEQ ID NO:55), ARFKVWWAAG (SEQ ID NO:56), SAFKVWWAAG (SEQ ID NO:57), SRFKAWWAAG (SEQ ID NO:60), SRFKVAWAAG (SEQ ID NO:61), SRFKVWAAAG (SEQ ID NO:62), SRFKVWWAAG (SEQ ID NO:63), SRFKVWWAAG (SEQ ID NO:64), RFKVWWAAG (SEQ ID NO:65), SRFKVWWAG (SEQ ID NO:68), SRFKVWWG (SEQ ID NO:69), or from SRFKVWWG (SEQ ID NO:73), SRFKVWWG (SEQ ID NO:74), ARFKVWWG (SEQ ID NO:75), ARFKVWWG (SEQ ID NO:76), ARFKVWWGG (SEQ ID NO:77), ARFKVWWGG (SEQ ID NO:78), ARFKVWWA (SEQ ID NO:79), ARFKVWWA (SEQ ID NO:80), ARFKVWWAA (SEQ ID NO:81) and ARFKVWWA(CONH₂) (SEQ ID NO:82), or an amino acid sequence, which is SRFKVWWAAG (SEQ ID NO:1. The novel peptides are capable of binding to human kallikrein 2 and of inhibiting the proteolytic activity of human kallikrein 2. They can be used in prostate cancer treatment as such or in combination with other ligands modulating the activity of factors mediating tumor growth.

SEQUENCE LISTING

<110> Licentia Ltd.

<120> Novel peptides

<130> LIC7

<160> 130

<170> PatentIn version 3.3

<210> 1
<211> 10
<212> PRT
<213> artificial sequence

<220>
<223> synthetic peptide

<400> 1

Ser Arg Phe Lys Val Trp Trp Ala Ala Gly
1               5                   10

<210> 2
<211> 10
<212> PRT
<213> artificial sequence

<220>
<223> synthetic peptide

<400> 2

Ala Ala Arg Arg Pro Phe Pro Ala Pro Ser
1               5                   10

<210> 3
<211> 10
<212> PRT
<213> artificial sequence

<220>
<223> synthetic peptide

<400> 3

Pro Ala Arg Arg Pro Phe Pro Val Thr Ala
1               5                   10

<210> 4
<211> 10
<212> PRT
<213> artificial sequence

<220>
<223> synthetic peptide

<400> 4

Cys Phe Arg Gln Gly Cys Trp Val Ile Thr
1               5                   10

<210> 5
<211> 11
<212> PRT
<213> artificial sequence

<220>
<223> synthetic peptide

<400> 5

Phe Arg Thr Cys Leu Arg Ser Trp Ala Cys Met
1               5                   10

<210> 6
<211> 11
<212> PRT
<213> artificial sequence

<220>
<223>  synthetic peptide

<400>  6

Cys Tyr Arg Met Pro Thr Cys Met Gln Arg Asp
1               5                   10


<210>  7
<211>  12
<212>  PRT
<213>  artificial sequence

<220>
<223>  synthetic peptide

<400>  7

Gly Ala Ala Arg Arg Pro Phe Pro Ala Pro Ser Gly
1               5                   10


<210>  8
<211>  12
<212>  PRT
<213>  artificial sequence

<220>
<223>  synthetic peptide

<400>  8

Gly Ala Ala Ala Arg Pro Phe Pro Ala Pro Ser Gly
1               5                   10


<210>  9
<211>  12
<212>  PRT
<213>  artificial sequence

<220>
<223>  synthetic peptide

<400>  9

Gly Ala Ala Arg Ala Pro Phe Pro Ala Pro Ser Gly
1               5                   10


<210>  10
<211>  12
<212>  PRT
<213>  artificial sequence

<220>
<223>  synthetic peptide

<400>  10

Gly Ala Ala Arg Arg Ala Phe Pro Ala Pro Ser Gly
1               5                   10


<210>  11
<211>  12
<212>  PRT
<213>  artificial sequence

<220>
<223>  synthetic peptide

<400>  11

Gly Ala Ala Arg Arg Pro Ala Pro Ala Pro Ser Gly
1               5                   10


<210>  12
<211>  12
<212>  PRT
<213>  artificial sequence

<220>

<223>  synthetic peptide

<400>  12

Gly Ala Ala Arg Arg Pro Phe Ala Ala Pro Ser Gly
1               5                   10


<210>  13
<211>  12
<212>  PRT
<213>  artificial sequence

<220>
<223>  synthetic peptide

<400>  13

Gly Ala Ala Arg Arg Pro Phe Pro Ala Ala Ser Gly
1               5                   10


<210>  14
<211>  12
<212>  PRT
<213>  artificial sequence

<220>
<223>  synthetic peptide

<400>  14

Gly Ala Ala Arg Arg Pro Phe Pro Ala Pro Ala Gly
1               5                   10


<210>  15
<211>  12
<212>  PRT
<213>  artificial sequence

<220>
<223>  synthetic peptide

<400>  15

Gly Pro Ala Arg Arg Pro Phe Pro Val Thr Ala Gly
1               5                   10


<210>  16
<211>  12
<212>  PRT
<213>  artificial sequence

<220>
<223>  synthetic peptide

<400>  16

Gly Ala Ala Arg Arg Pro Phe Pro Val Thr Ala Gly
1               5                   10


<210>  17
<211>  12
<212>  PRT
<213>  artificial sequence

<220>
<223>  synthetic peptide

<400>  17

Gly Pro Ala Ala Arg Pro Phe Pro Val Thr Ala Gly
1               5                   10


<210>  18
<211>  12
<212>  PRT
<213>  artificial sequence

<220>
<223>  synthetic peptide

<400>  18

Gly Pro Ala Arg Ala Pro Phe Pro Val Thr Ala Gly
1               5                   10


<210>  19
<211>  12
<212>  PRT
<213>  artificial sequence

<220>
<223>  synthetic peptide

<400>  19

Gly Pro Ala Arg Arg Ala Phe Pro Val Thr Ala Gly
1               5                   10


<210>  20
<211>  12
<212>  PRT
<213>  artificial sequence

<220>
<223>  synthetic peptide

<400>  20

Gly Pro Ala Arg Arg Pro Ala Pro Val Thr Ala Gly
1               5                   10


<210>  21
<211>  12
<212>  PRT
<213>  artificial sequence

<220>
<223>  synthetic peptide

<400>  21

Gly Pro Ala Arg Arg Pro Phe Ala Val Thr Ala Gly
1               5                   10


<210>  22
<211>  12
<212>  PRT
<213>  artificial sequence

<220>
<223>  synthetic peptide

<400>  22

Gly Pro Ala Arg Arg Pro Phe Pro Ala Thr Ala Gly
1               5                   10


<210>  23
<211>  12
<212>  PRT
<213>  artificial sequence

<220>
<223>  synthetic peptide

<400>  23

Gly Pro Ala Arg Arg Pro Phe Pro Val Ala Ala Gly
1               5                   10


<210>  24
<211>  11
<212>  PRT
<213>  artificial sequence

<220>
<223>  synthetic peptide

<400>  24

Gly Pro Ala Arg Arg Pro Phe Pro Val Thr Gly
1               5                   10

<210>  25
<211>  10
<212>  PRT
<213>  artificial sequence

<220>
<223>  synthetic peptide

<400>  25

Gly Pro Ala Arg Arg Pro Phe Pro Val Gly
1               5                   10

<210>  26
<211>  9
<212>  PRT
<213>  artificial sequence

<220>
<223>  synthetic peptide

<400>  26

Gly Pro Ala Arg Arg Pro Phe Pro Gly
1               5

<210>  27
<211>  8
<212>  PRT
<213>  artificial sequence

<220>
<223>  synthetic peptide

<400>  27

Gly Pro Ala Arg Arg Pro Phe Gly
1               5

<210>  28
<211>  7
<212>  PRT
<213>  artificial sequence

<220>
<223>  synthetic peptide

<400>  28

Gly Pro Ala Arg Arg Pro Gly
1               5

<210>  29
<211>  6
<212>  PRT
<213>  artificial sequence

<220>
<223>  synthetic peptide

<400>  29

Gly Pro Ala Arg Arg Gly
1               5

<210>  30
<211>  5
<212>  PRT
<213>  artificial sequence

<220>
<223>  synthetic peptide

<400>  30

Gly Pro Ala Arg Gly
1               5

<210>  31
<211>  12
<212>  PRT
<213>  artificial sequence

<220>
<223>  synthetic peptide

<400>  31

Gly Pro Ala Arg Arg Pro Phe Pro Val Thr Ala Gly
1               5                   10


<210>  32
<211>  11
<212>  PRT
<213>  artificial sequence

<220>
<223>  synthetic peptide

<400>  32

Gly Ala Arg Arg Pro Phe Pro Val Thr Ala Gly
1               5                   10


<210>  33
<211>  10
<212>  PRT
<213>  artificial sequence

<220>
<223>  synthetic peptide

<400>  33

Gly Arg Arg Pro Phe Pro Val Thr Ala Gly
1               5                   10


<210>  34
<211>  9
<212>  PRT
<213>  artificial sequence

<220>
<223>  synthetic peptide

<400>  34

Gly Arg Pro Phe Pro Val Thr Ala Gly
1               5


<210>  35
<211>  8
<212>  PRT
<213>  artificial sequence

<220>
<223>  synthetic peptide

<400>  35

Gly Pro Phe Pro Val Thr Ala Gly
1               5


<210>  36
<211>  7
<212>  PRT
<213>  artificial sequence

<220>
<223>  synthetic peptide

<400>  36

Gly Phe Pro Val Thr Ala Gly
1               5

<210>  37
<211>  6
<212>  PRT
<213>  artificial sequence

<220>
<223>  synthetic peptide

<400>  37

Gly Pro Val Thr Ala Gly
1               5


<210>  38
<211>  5
<212>  PRT
<213>  artificial sequence

<220>
<223>  synthetic peptide

<400>  38

Gly Val Thr Ala Gly
1               5


<210>  39
<211>  5
<212>  PRT
<213>  artificial sequence

<220>
<223>  synthetic peptide

<400>  39

Arg Arg Pro Ala Pro
1               5


<210>  40
<211>  5
<212>  PRT
<213>  artificial sequence

<220>
<223>  synthetic peptide

<400>  40

Arg Arg Pro Phe Pro
1               5


<210>  41
<211>  5
<212>  PRT
<213>  artificial sequence

<220>
<223>  synthetic peptide

<400>  41

Arg Arg Pro Ala Gly
1               5


<210>  42
<211>  5
<212>  PRT
<213>  artificial sequence

<220>
<223>  synthetic peptide

<400>  42

Arg Arg Pro Phe Gly
1               5


<210>  43
<211>  7

<212>  PRT
<213>  artificial sequence

<220>
<223>  synthetic peptide

<400>  43

Gly Arg Arg Pro Ala Pro Gly
1               5


<210>  44
<211>  7
<212>  PRT
<213>  artificial sequence

<220>
<223>  synthetic peptide

<400>  44

Gly Arg Arg Pro Phe Pro Gly
1               5


<210>  45
<211>  8
<212>  PRT
<213>  artificial sequence

<220>
<223>  synthetic peptide

<400>  45

Ala Ala Arg Arg Pro Ala Pro Gly
1               5


<210>  46
<211>  9
<212>  PRT
<213>  artificial sequence

<220>
<223>  synthetic peptide

<400>  46

Ala Ala Ala Arg Arg Pro Ala Pro Gly
1               5


<210>  47
<211>  8
<212>  PRT
<213>  artificial sequence

<220>
<223>  synthetic peptide

<400>  47

Ala Ala Arg Arg Pro Phe Pro Gly
1               5


<210>  48
<211>  9
<212>  PRT
<213>  artificial sequence

<220>
<223>  synthetic peptide

<400>  48

Ala Ala Ala Arg Arg Pro Phe Pro Gly
1               5


<210>  49
<211>  11
<212>  PRT
<213>  artificial sequence

<220>
<223>  synthetic peptide


<220>
<221>  MISC_FEATURE
<222>  (1)..(11)
<223>  Xaa is AC

<400>  49

Xaa Cys Ala Ala Ala Arg Arg Pro Ala Pro Ala
1               5                   10


<210>  50
<211>  11
<212>  PRT
<213>  artificial sequence

<220>
<223>  synthetic peptide


<220>
<221>  MISC_FEATURE
<222>  (1)..(11)
<223>  Xaa is AC

<400>  50

Xaa Cys Ala Ala Ala Arg Arg Pro Ala Pro Gly
1               5                   10


<210>  51
<211>  11
<212>  PRT
<213>  artificial sequence

<220>
<223>  synthetic peptide

<400>  51

Gly Ala Ala Arg Arg Pro Phe Pro Ala Pro Gly
1               5                   10


<210>  52
<211>  11
<212>  PRT
<213>  artificial sequence

<220>
<223>  synthetic peptide


<220>
<221>  MISC_FEATURE
<222>  (1)..(11)
<223>  Xaa is Sar

<400>  52

Gly Ala Ala Arg Arg Xaa Phe Pro Ala Pro Gly
1               5                   10


<210>  53
<211>  11
<212>  PRT
<213>  artificial sequence

<220>
<223>  synthetic peptide


<220>
<221>  MISC_FEATURE
<222>  (1)..(11)
<223>  Xaa is Sar

<400>  53

Gly Ala Ala Arg Arg Pro Phe Xaa Ala Pro Gly
1               5                   10

<210>  54
<211>  11
<212>  PRT
<213>  artificial sequence

<220>
<223>  synthetic peptide

<220>
<221>  MISC_FEATURE
<222>  (1)..(11)
<223>  Xaa is SAR

<400>  54

Gly Ala Ala Arg Arg Xaa Phe Xaa Ala Pro Gly
1               5                   10

<210>  55
<211>  10
<212>  PRT
<213>  artificial sequence

<220>
<223>  synthetic peptide

<400>  55

Ser Arg Phe Lys Val Trp Trp Ala Ala Gly
1               5                   10

<210>  56
<211>  10
<212>  PRT
<213>  artificial sequence

<220>
<223>  synthetic peptide

<400>  56

Ala Arg Phe Lys Val Trp Trp Ala Ala Gly
1               5                   10

<210>  57
<211>  10
<212>  PRT
<213>  artificial sequence

<220>
<223>  synthetic peptide

<400>  57

Ser Arg Phe Lys Val Trp Trp Ala Ala Gly
1               5                   10

<210>  58
<211>  10
<212>  PRT
<213>  artificial sequence

<220>
<223>  synthetic peptide

<400>  58

Ser Arg Ala Lys Val Trp Trp Ala Ala Gly
1               5                   10

<210>  59
<211>  10
<212>  PRT
<213>  artificial sequence

<220>

<223>  synthetic peptide

<400>  59

Ser Arg Phe Ala Val Trp Trp Ala Ala Gly
1               5                   10


<210>  60
<211>  10
<212>  PRT
<213>  artificial sequence

<220>
<223>  synthetic peptide

<400>  60

Ser Arg Phe Lys Ala Trp Trp Ala Ala Gly
1               5                   10


<210>  61
<211>  10
<212>  PRT
<213>  artificial sequence

<220>
<223>  synthetic peptide

<400>  61

Ser Arg Phe Lys Val Ala Trp Ala Ala Gly
1               5                   10


<210>  62
<211>  10
<212>  PRT
<213>  artificial sequence

<220>
<223>  synthetic peptide

<400>  62

Ser Arg Phe Lys Val Trp Ala Ala Ala Gly
1               5                   10


<210>  63
<211>  10
<212>  PRT
<213>  artificial sequence

<220>
<223>  synthetic peptide

<400>  63

Ser Arg Phe Lys Val Trp Trp Ala Ala Gly
1               5                   10


<210>  64
<211>  10
<212>  PRT
<213>  artificial sequence

<220>
<223>  synthetic peptide

<400>  64

Ser Arg Phe Lys Val Trp Trp Ala Ala Gly
1               5                   10


<210>  65
<211>  9
<212>  PRT
<213>  artificial sequence.

<220>
<223>  synthetic peptide

<400> 65

Arg Phe Lys Val Trp Trp Ala Ala Gly
1               5


<210> 66
<211> 8
<212> PRT
<213> artificial sequence

<220>
<223> synthetic peptide

<400> 66

Phe Lys Val Trp Trp Ala Ala Gly
1               5


<210> 67
<211> 5
<212> PRT
<213> artificial sequence

<220>
<223> synthetic peptide

<400> 67

Trp Trp Ala Ala Gly
1               5


<210> 68
<211> 9
<212> PRT
<213> artificial sequence

<220>
<223> synthetic peptide

<400> 68

Ser Arg Phe Lys Val Trp Trp Ala Gly
1               5


<210> 69
<211> 8
<212> PRT
<213> artificial sequence

<220>
<223> synthetic peptide

<400> 69

Ser Arg Phe Lys Val Trp Trp Gly
1               5


<210> 70
<211> 7
<212> PRT
<213> artificial sequence

<220>
<223> synthetic peptide

<400> 70

Ser Arg Phe Lys Val Trp Gly
1               5


<210> 71
<211> 6
<212> PRT
<213> artificial sequence

<220>
<223> synthetic peptide

<400> 71

Ser Arg Phe Lys Val Gly
1               5

<210>  72
<211>  5
<212>  PRT
<213>  artificial sequence

<220>
<223>  synthetic peptide

<400>  72

Ser Arg Phe Lys Gly
1               5

<210>  73
<211>  8
<212>  PRT
<213>  artificial sequence

<220>
<223>  synthetic peptide

<400>  73

Ser Arg Phe Lys Val Trp Trp Gly
1               5

<210>  74
<211>  9
<212>  PRT
<213>  artificial sequence

<220>
<223>  synthetic peptide

<220>
<221>  MISC_FEATURE
<222>  (1)..(9)
<223>  Xaa is AC

<400>  74

Xaa Ser Arg Phe Lys Val Trp Trp Gly
1               5

<210>  75
<211>  8
<212>  PRT
<213>  artificial sequence

<220>
<223>  synthetic peptide

<400>  75

Ala Arg Phe Lys Val Trp Trp Gly
1               5

<210>  76
<211>  9
<212>  PRT
<213>  artificial sequence

<220>
<223>  synthetic peptide

<220>
<221>  MISC_FEATURE
<222>  (1)..(9)
<223>  Xaa is AC

<400>  76

Xaa Ala Arg Phe Lys Val Trp Trp Gly
1               5

<210>  77
<211>  10
<212>  PRT
<213>  artificial sequence

<220>
<223>  synthetic peptide


<220>
<221>  MISC_FEATURE
<222>  (1)..(10)
<223>  Xaa is AC

<400>  77

Xaa Ala Arg Phe Lys Val Trp Trp Gly Gly
1               5                   10


<210>  78
<211>  9
<212>  PRT
<213>  artificial sequence

<220>
<223>  synthetic peptide

<400>  78

Ala Arg Phe Lys Val Trp Trp Gly Gly
1               5


<210>  79
<211>  8
<212>  PRT
<213>  artificial sequence

<220>
<223>  synthetic peptide

<400>  79

Ala Arg Phe Lys Val Trp Trp Ala
1               5


<210>  80
<211>  9
<212>  PRT
<213>  artificial sequence

<220>
<223>  synthetic peptide


<220>
<221>  MISC_FEATURE
<222>  (1)..(9)
<223>  Xaa is AC

<400>  80

Xaa Ala Arg Phe Lys Val Trp Trp Ala
1               5


<210>  81
<211>  10
<212>  PRT
<213>  artificial sequence

<220>
<223>  synthetic peptide


<220>
<221>  MISC_FEATURE
<222>  (1)..(10)
<223>  Xaa is AC

<400>  81

Xaa Ala Arg Phe Lys Val Trp Trp Ala Ala
1               5                   10

<210>  82
<211>  9
<212>  PRT
<213>  artificial sequence

<220>
<223>  synthetic peptide


<220>
<221>  MISC_FEATURE
<222>  (1)..(10)
<223>  Xaa is CONH2

<400>  82

Ala Arg Phe Lys Val Trp Trp Ala Xaa
1               5


<210>  83
<211>  42
<212>  DNA
<213>  artificial sequence

<220>
<223>  primer

<400>  83
taatacgact cactataggg caagctgata aaccgataca at                          42


<210>  84
<211>  14
<212>  PRT
<213>  artificial sequence

<220>
<223>  synthetic peptide


<220>
<221>  misc_feature
<222>  (1)..(14)
<223>  Xaa is Cystein with or wothout Acm protection group

<400>  84

Gly Xaa Ala Ala Arg Phe Lys Val Trp Trp Ala Ala Cys Gly
1               5                   10


<210>  85
<211>  11
<212>  PRT
<213>  artificial sequence

<220>
<223>  synthetic peptide


<220>
<221>  MISC_FEATURE
<222>  (1)..(11)
<223>  Xaa is Cystein with Acm protection group

<400>  85

Xaa Ala Arg Arg Pro Ala Pro Ala Pro Cys Gly
1               5                   10


<210>  86
<211>  12
<212>  PRT
<213>  artificial sequence

<220>
<223>  synthetic peptide


<220>
<221>  MISC_FEATURE
<222>  (1)..(12)

<223>  Xaa is Cystein with Acm protection group

<400>  86

Xaa Ala Ala Arg Arg Pro Ala Pro Ala Pro Cys Gly
1               5                   10


<210>  87
<211>  13
<212>  PRT
<213>  artificial sequence

<220>
<223>  synthetic peptide


<220>
<221>  MISC_FEATURE
<222>  (1)..(13)
<223>  Xaa is Cystein with Acm protection group

<400>  87

Xaa Ala Ala Ala Arg Arg Pro Ala Pro Ala Pro Cys Gly
1               5                   10


<210>  88
<211>  9
<212>  PRT
<213>  artificial sequence

<220>
<223>  synthetic peptide


<220>
<221>  MISC_FEATURE
<222>  (1)..(9)
<223>  Xaa is Cystein with Acm protection group

<400>  88

Xaa Arg Arg Pro Ala Pro Ala Cys Gly
1               5


<210>  89
<211>  10
<212>  PRT
<213>  artificial sequence

<220>
<223>  synthetic peptide


<220>
<221>  MISC_FEATURE
<222>  (1)..(10)
<223>  Xaa is Cystein with Acm protection group

<400>  89

Xaa Ala Arg Arg Pro Ala Pro Ala Cys Gly
1               5                   10


<210>  90
<211>  11
<212>  PRT
<213>  artificial sequence

<220>
<223>  synthetic peptide


<220>
<221>  MISC_FEATURE
<222>  (1)..(11)
<223>  Xaa is Cystein with Acm protection group

<400>  90

Xaa Ala Arg Arg Pro Ala Pro Ala Ala Cys Gly
1               5                   10

<210> 91
<211> 12
<212> PRT
<213> artificial sequence

<220>
<223> synthetic peptide


<220>
<221> MISC_FEATURE
<222> (1)..(12)
<223> Xaa is Cystein with Acm protection group

<400> 91

Xaa Ala Ala Arg Arg Pro Ala Pro Ala Ala Cys Gly
1               5                   10


<210> 92
<211> 13
<212> PRT
<213> artificial sequence

<220>
<223> synthetic peptide


<220>
<221> MISC_FEATURE
<222> (1)..(13)
<223> Xaa is Cystein with Acm protection group

<400> 92

Xaa Ala Ala Ala Arg Arg Pro Ala Pro Ala Ala Cys Gly
1               5                   10


<210> 93
<211> 5
<212> PRT
<213> artificial sequence

<220>
<223> synthetic peptide


<220>
<221> MISC_FEATURE
<222> (1)..(5)
<223> Xaa is any amino acid

<400> 93

Arg Arg Pro Xaa Pro
1               5


<210> 94
<211> 6
<212> PRT
<213> artificial sequence

<220>
<223> synthetic peptide


<220>
<221> MISC_FEATURE
<222> (1)..(6)
<223> Xaa is any amino acid

<400> 94

Ala Arg Arg Pro Xaa Pro
1               5


<210> 95
<211> 6
<212> PRT
<213> artificial sequence

<220>
<223> synthetic peptide

<400> 95

Ala Arg Arg Pro Phe Pro
1               5


<210> 96
<211> 5
<212> PRT
<213> artificial sequence

<220>
<223> synthetic peptide


<220>
<221> MISC_FEATURE
<222> (1)..(5)
<223> Xaa is any amino acid

<400> 96

Arg Phe Lys Xaa Trp
1               5


<210> 97
<211> 5
<212> PRT
<213> artificial sequence

<220>
<223> synthetic peptide

<400> 97

Arg Phe Lys Val Trp
1               5


<210> 98
<211> 7
<212> PRT
<213> artificial sequence

<220>
<223> synthetic peptide

<400> 98

Ala Arg Phe Lys Val Trp Trp
1               5


<210> 99
<211> 4
<212> PRT
<213> artificial sequence

<220>
<223> synthetic peptide

<400> 99

Pro Ala Pro Ser
1


<210> 100
<211> 4
<212> PRT
<213> artificial sequence

<220>
<223> synthetic peptide

<400> 100

Ala Ala Pro Ser
1

<210>  101
<211>  4
<212>  PRT
<213>  artificial sequence

<220>
<223>  synthetic peptide

<400>  101

Pro Ala Ala Ser
1


<210>  102
<211>  4
<212>  PRT
<213>  artificial sequence

<220>
<223>  synthetic peptide

<400>  102

Pro Ala Pro Ala
1


<210>  103
<211>  4
<212>  PRT
<213>  artificial sequence

<220>
<223>  synthetic peptide

<400>  103

Pro Val Thr Ala
1


<210>  104
<211>  4
<212>  PRT
<213>  artificial sequence

<220>
<223>  synthetic peptide

<400>  104

Pro Ala Thr Ala
1


<210>  105
<211>  4
<212>  PRT
<213>  artificial sequence

<220>
<223>  synthetic peptide

<400>  105

Pro Val Ala Ala
1


<210>  106
<211>  3
<212>  PRT
<213>  artificial sequence

<220>
<223>  synthetic peptide

<400>  106

Pro Val Thr
1


<210>  107
<211>  2

<212>  PRT
<213>  artificial sequence

<220>
<223>  synthetic peptide

<400>  107

Pro Val
1


<210>  108
<211>  2
<212>  PRT
<213>  artificial sequence

<220>
<223>  synthetic peptide

<400>  108

Pro Ala
1


<210>  109
<211>  3
<212>  PRT
<213>  artificial sequence

<220>
<223>  synthetic peptide

<400>  109

Pro Ala Pro
1


<210>  110
<211>  3
<212>  PRT
<213>  artificial sequence

<220>
<223>  synthetic peptide


<220>
<221>  MISC_FEATURE
<222>  (1)..(3)
<223>  X is Sar

<400>  110

Xaa Ala Pro
1


<210>  111
<211>  5
<212>  PRT
<213>  artificial sequence

<220>
<223>  synthetic peptide

<400>  111

Val Trp Trp Ala Ala
1               5


<210>  112
<211>  5
<212>  PRT
<213>  artificial sequence

<220>
<223>  synthetic peptide

<400>  112

Ala Trp Trp Ala Ala
1               5

<210> 113
<211> 5
<212> PRT
<213> artificial sequence

<220>
<223> synthetic peptide

<400> 113

Val Ala Trp Ala Ala
1               5


<210> 114
<211> 5
<212> PRT
<213> artificial sequence

<220>
<223> synthetic peptide

<400> 114

Val Trp Ala Ala Ala
1               5


<210> 115
<211> 4
<212> PRT
<213> artificial sequence

<220>
<223> synthetic peptide

<400> 115

Val Trp Trp Ala
1


<210> 116
<211> 3
<212> PRT
<213> artificial sequence

<220>
<223> synthetic peptide

<400> 116

Val Trp Trp
1


<210> 117
<211> 2
<212> PRT
<213> artificial sequence

<220>
<223> synthetic peptide

<400> 117

Val Trp
1


<210> 118
<211> 2
<212> PRT
<213> artificial sequence

<220>
<223> synthetic peptide

<400> 118

Val Gly
1

<210> 119
<211> 4
<212> PRT
<213> artificial sequence

<220>
<223> synthetic peptide

<400> 119

Val Trp Trp Gly
1


<210> 120
<211> 5
<212> PRT
<213> artificial sequence

<220>
<223> synthetic peptide

<400> 120

Val Trp Trp Ala Ala
1               5


<210> 121
<211> 3
<212> PRT
<213> artificial sequence

<220>
<223> synthetic peptide

<400> 121

Gly Ala Ala
1


<210> 122
<211> 3
<212> PRT
<213> artificial sequence

<220>
<223> synthetic peptide

<400> 122

Gly Pro Ala
1


<210> 123
<211> 2
<212> PRT
<213> artificial sequence

<220>
<223> synthetic peptide

<400> 123

Gly Ala
1


<210> 124
<211> 2
<212> PRT
<213> artificial sequence

<220>
<223> synthetic peptide

<400> 124

Ala Ala
1


<210> 125
<211> 3

<212>  PRT
<213>  artificial sequence

<220>
<223>  synthetic peptide

<400>  125

Ala Ala Ala
1


<210>  126
<211>  3
<212>  PRT
<213>  artificial sequence

<220>
<223>  synthetic peptide

<400>  126

Ala Ala Ala
1


<210>  127
<211>  6
<212>  PRT
<213>  artificial sequence

<220>
<223>  synthetic peptide


<220>
<221>  MISC_FEATURE
<222>  (1)..(6)
<223>  Xaa is any amino acid

<400>  127

Ala Arg Phe Lys Xaa Trp
1               5


<210>  128
<211>  12
<212>  PRT
<213>  artificial sequence

<220>
<223>  synthetic peptide


<220>
<221>  misc_feature
<222>  (1)..(12)
<223>  Xaa is Cysteine with or without Acm protective group

<400>  128

Gly Xaa Ala Arg Phe Lys Val Trp Trp Ala Cys Gly
1               5                   10


<210>  129
<211>  10
<212>  PRT
<213>  artificial sequence

<220>
<223>  synthetic peptide


<220>
<221>  misc_feature
<222>  (1)..(10)
<223>  Xaa is Cystein with or without Acm protection group

<400>  129

Gly Xaa Arg Phe Lys Val Trp Trp Cys Gly
1               5                   10

<210> 130
<211> 42
<212> DNA
<213> artificial sequence

<220>
<223> primer

<400> 130
taatacgact cactataggg caagctgata aaccgataca at 42

## Description

### Background of the Invention

### Field of the Invention

The present invention relates to novel peptides, which bind to human kallikrein and are capable of modulating its enzyme activity. In particular, the present invention concerns novel peptides, which are useful as inhibitors of human kallikrein 2 (hK2). The present invention also relates to a process for the preparation of these peptides. Further, the present invention concerns pharmaceutical and diagnostic compositions comprising these peptides and the use of the peptides for pharmaceutical and diagnostic preparations. Still further, the present invention relates to the use of these peptides as medicaments and diagnostic agents, for the preparation of medicaments and diagnostic agents and in biochemical isolation and purification procedures.

### Description of Related Art

Human kallikrein 2 (hK2) is a serine protease produced by the secretory epithelial cells in the prostate. Because hK2 activates several factors participating in proteolytic cascades that may mediate metastasis of prostate cancer, modulation of the activity of hK2 is a potential way of preventing tumor growth and metastasis. Furthermore, specific ligands for hK2 are potentially useful for targeting and imaging of prostate cancer.

Prostate cancer is the most common non-skin cancer of males in industrialized countries. Screening based on determination of serum PSA can be used to detect early prostate cancer, which, when localized to the prostate, is potentially curable by radical prostatectomy or radiotherapy (Chodak et al. 1994). However, 20 ― 30 % of cancers detected by screening show extra-prostatic spread and will eventually develop into an advanced disease, which at present cannot be cured. It is therefore very important to identify new therapeutic agents, which could slow down the growth of the small tumors so that clinical disease would not develop during the life-time of the patient. Advanced disease can be treated by endocrine therapy, but androgen-resistance usually develops within 2-5 years, for which there is no effective treatment (Denis and Griffiths 2000). Thus, novel pharmaceuticals need to be developed for treating both localized and metastatic prostate cancer.

The prostate produces several proteases that are secreted into seminal fluid. Two of these have been shown to be highly prostate specific, i.e., prostate specific antigen (PSA) and hK2 (Wang et al. 1979; Chapdelaine et al. 1988; Morris 1989). Even though PSA is a sensitive serum marker of prostate cancer, its expression is actually lower in malignant than in normal prostatic epithelium and it is further reduced in poorly differentiated tumors (Stege et al. 2000). By contrast, the expression of hK2 is increased in aggressive tumors (Darson et al. 1997). hK2 has been shown to activate the proforms of PSA and urokinase type plasminogen activator (uPA) (Frenette et al. 1997) and to inactivate plasminogen activator inhibitor-1 (PAI-1)(Mikolajczyk et al. 1999), suggesting a possible role in a proteolytic cascade associated with prostate cancer. Thus, hK2 may act as a promoter of tumor growth and metastasis and inhibition of hK2 activity may therefore slow down tumor spread. Because hK2 is highly prostate-specific, inhibition of its activity is a potential way of preventing prostate cancer growth and metastasis without side effects outside the prostate.

Peptides are a potential alternative to monoclonal antibodies for treatment (Pasqualini et al. 1997) and diagnostic applications (Wu et al. 2004). Screening of random peptide libraries has led to development of novel types of enzyme inhibitors, peptide substrates and ligands binding to the active site of receptors (Ruoslahti and Rajotte 2000). Recently developed peptide ligands to PSA show a novel type of binding specificity in the sense that they only react with the enzymatically active form of free PSA (Wu et al. 2000; Wu et al. 2004). By using these peptides a novel type of assay, called immunopeptidometric assay (IPMA), could be developed (Wu, Zhu et al. 2004). Thus, small peptides, which act against prostate specific proteinases, are potentially useful ligands for development of novel diagnostic assays, but perhaps more importantly, they can be used for targeting of prostatic tumors. Because of their small size, peptides are not immunogenic and show faster tissue penetration than antibodies; they are easy to synthesize chemically and can be derivatized in several ways for labeling with isotopes (Liu and Edwards 1999),conjugation with toxins (Denmeade et al. 1998) or for modification of pharmacokinetics (Morpurgo et al. 2002). Peptides modulating the enzyme activity of proteases may inhibit the growth of cancer (Koivunen et al. 1999; Wu, Leinonen et al. 2000). Radioactively labeled peptides are potentially useful for radioimaging and peptide conjugates with cytotoxic drugs or boron compounds can be used for killing of tumor cells (Boerman et al. 2000).

In the art, no peptides have been suggested which would be capable of inhibiting the activity of hK2.

### Summary of the Invention

It is an object of the present invention to eliminate problems of the prior art and to provide novel hK2 binding chemical agents. In particular, the invention aims at providing novel peptide ligands and functional equivalents thereof, which are therapeutically and diagnostically useful in particularly for treatment and diagnosis of conditions involving release of hK2 into circulation.

It is another object to provide a process for the preparation of novel peptide ligands for human kallikrein 2, which are capable of inhibiting its enzymatic activity.

It is a third object to provide pharmaceutical and diagnostic compositions comprising novel chemical compounds capable of binding to hK2.

Further, it is a fourth object of the invention to provide novel diagnostic and therapeutical methods.

These and other objects, together with the advantages thereof over known peptides and processes, are achieved by the present invention as hereinafter described and claimed.

The present invention is based on the finding that a group of novel peptides having a specific structure (specific amino acid sequences or motifs) are capable of selectively binding to human kallikrein 2 and of inhibiting its enzymatic activity.

In the course of the present invention, screening of phage display peptide libraries with recombinant hK2 resulted in the isolation of several hK2-binding peptides, which were efficient and specific inhibitors of hK2. The motifs required for inhibitory activity were characterized by amino acid substitution analysis, which also has led to identification of peptides with improved inhibitory potency. We have previously identified specific PSA-binding peptides by using the same methodology (Wu, Leinonen et al. 2000), but contrary to the hK2-inhibiting peptides, the PSA-binding peptides enhanced the enzyme activity of PSA.

Thus, according to the invention, novel isolated peptides are provided having the amino acid sequence of Formula I

X¹ X² X³ X⁴, I

wherein
- X¹: is an optional residue of an amino acid selected from the group of R, F, Y, S, A and conservative substitutes thereof;
- X²: stands for a residue of an amino acid selected from the group of R and A and conservative substitutes thereof;
- X³: is a residue of an amino acid selected from the group of F, P, G, Q, M, R and conservative substitutes thereof; and
- X⁴: is a residue of an amino acid selected from the group of K, F, G, P and conservative substitutes thereof.

These peptides are capable of binding to human kallikrein 2 and, simultaneously, of inhibiting the proteolytic activity of human kallikrein 2.

The present binding and inhibiting peptides provide for a number of therapeutic working embodiments. Thus, according to one such interesting embodiment, a method is provided for inhibiting prostate tumour growth and/or spread, which method comprises contacting a prostate cell with a nucleic acid - comprising a nucleotide sequence encoding the peptide ― and further comprising a promoter active in said cell. The promoter is operably linked to the nucleotide sequence encoding said peptide, and the contacting takes place under conditions permitting the uptake of said nucleic acid and expression of the peptide by the cell in an amount effective to inhibit growth and/or spread of said cell.

Other therapeutic and diagnostic embodiments are described in more detail below.

The present peptides can also be used as lead compounds in methods of designing peptidomimetic compounds.

More specifically, the peptide ligands and functional equivalents thereof according to the present invention are mainly characterized by what is stated in claims 1, 25 and 28.

The methods of using the peptides are characterized by what is stated in the characterizing parts of claims 45, 47, 48, 51, 56 and 57.

The invention also provides novel nucleic acids according to claim 35 and their uses as claimed in claim 36.

Considerable advantages are obtained with the aid of the present invention. Prostate specific antigen (PSA) and human kallikrein 2 (hK2) show 80 % similarity in amino acid sequence and so far it has been difficult to develop specific antibody-based assays for hK2. The hK2-binding peptides according to the invention present a solution to this problem. Thus, small synthetic peptides can be used as tracers and polymeric peptide ligands, which through multivalent binding show significantly increased affinity to the target, may be prepared.

Further, there is evidence that hK2 expression is upregulated in aggressive prostate cancer and hK2 has been shown to activate several factors mediating cancer spread. The present peptides represent a new type of ligand for hK2. They have been shown to inhibit cleavage of a peptide substrate by hK2 and they are potentially useful agents for inhibiting growth and spread of prostate cancer.

Thus, the present peptides are expected to be useful in prostate cancer treatment as such or in combination with other ligands modulating the activity of factors mediating tumor growth.

Furthermore, the present peptides are potentially useful as radioactively, lanthanide or magnetic particle labeled derivates for tumor imaging with single photon emission computed tomography/positron emission tomography (SPECT/PET) or magnetic resonance imaging (MRI) or they may be conjugated with cytotoxic drugs to be used for targeted killing of the tumor cells. Exemplary cytotoxic agents include radioisotopes, any known anti-neoplastic drug or pro-drug that can be administered and converted to a drug *in vivo;* and the like. Alternatively, the peptide is embedded in a drug-containing liposome (e.g., with a hydrophobic tail) and used to target the liposome to hK2-expressing cells.

In addition to the foregoing, the invention includes, as an additional aspect, all embodiments of the invention narrower in scope in any way than the variations specifically mentioned above. Although the applicant(s) invented the full scope of the claims appended hereto, said claims are not intended to encompass within their scope the prior art works of others. Therefore, in the event that statutory prior art within the scope of a claim is brought to the attention of the applicants by a Patent Office or other entity or individual, the applicant(s) reserve the right to exercise amendment rights under applicable patent laws to redefine the subject matter of such a claim to specifically exclude such statutory prior art or obvious variations of statutory prior art from the scope of such a claim. Variations of the invention defined by such amended claims also are intended as aspects of the invention.

Next, the invention will be described in more detail with the aid of a detailed description and by making reference to the attached drawings.

### Brief Description of the Drawings

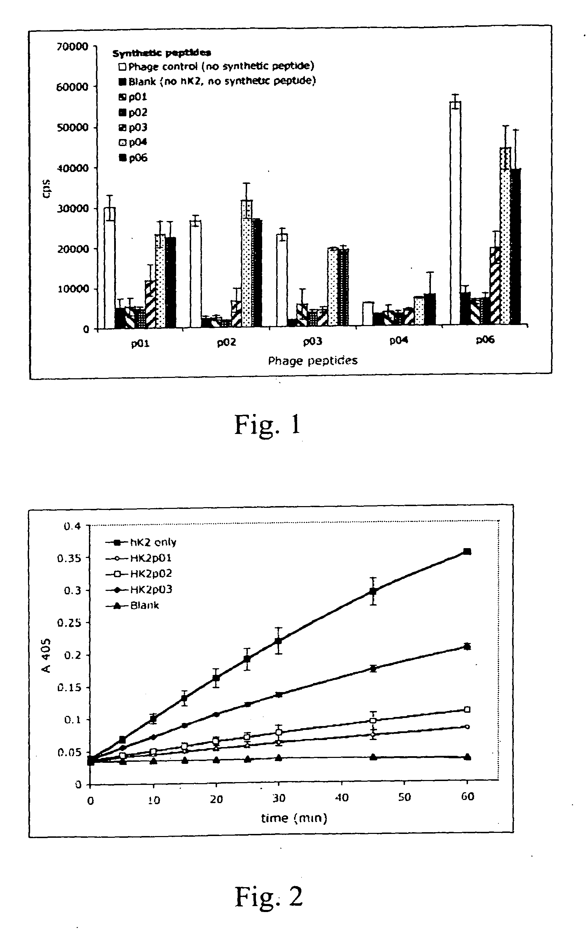

Figure 1 shows cross inhibition assays of hK2 binding peptides. Each of the synthetic peptides was incubated with hK2 captured by antibodies and further incubated with each of the phage peptides shown in the x-axis. The white bar represents binding of phage peptides with no preincubation of synthetic peptides acting as the control for the binding specificity, whereas the black bar shows non-specific binding acting as blank. The rest of the bars indicate the binding of the phage peptides after preincubation with each of the hK2 binding synthetic peptides. Bound phages were detected by measuring the fluorescence of europium labeled anti-phage polyclonal antibodies. Data represents mean values from duplicate wells ± standard error (SE).
Figure 2 depicts graphically the enzymatic activity of hK2 in the presence of hK2- inhibiting peptides from the X10 phage library. hK2 (1.7 µM) was incubated with the 1000-fold molar excess of hK2-inhibiting peptides for 30 min. After addition of chromogenic substrate the enzymatic activity was observed by measuring the absorbance at 405 nm. Data represents mean values from duplicate wells ± standard error (SE).
Figure 3 depicts the enzymatic activity of hK2 in the presence of various cyclic peptides.
Figure 4 shows the structure of DTPA labeled hK2- or PSA-specific peptides with a polyethylene spacer.

### Detailed Description of the Invention

### Definitions:

For the purpose of the present invention, the term "ligand" stands for a chemical compound or a part of a chemical compound, which is capable of binding to the binding domain present on a large polypeptide molecule, such as a hormone receptor or an enzyme. The present peptide ligands bind to hK2, and inhibit its enzyme activity. In the present context, "ligand" is used synonymously with "binding agent".

Generally, the ligand is a chemical compound, e.g. a peptide, a peptide analog or mimetic, which is soluble in physiological solutions or miscible with water and serum. The binding of the present ligands to hK2 can be characterized as being "stable" (in contrast to, e.g., an enzymatic reaction) in the respect that the ligand is attached to a binding domain of hK2 to the extent that its binding can be measured and determined e.g. by surface plasmon resonance or immuno-peptidometric assays (IPMA-assay). Further, the ligand remains bound during washing with physiologically buffered water. The bonding strength of the present ligands is comparable to that of peptide agents used for the targeting of breast cancer (Arap et al. 1998).

"Inhibition", as used in the present context, stands for an inhibitory action on the proteolytic activity of hK2. The inhibition of the peptides was compared by determining IC50 constants for hK2 inhibition, which were obtained by determining the velocity of the cleavage of S-2302 substrate (Chromogenix) at 200 µM concentration by 0.17 µM hK2 *at 0- 400 µM peptide* concentrations. In addition, the Ki values for the most potent inhibitory peptide were determined from Lineweaver-Burke plots after determining the velocity of the cleavage *of 0-400 µM* S-2302 substrate by 0.2 µM hK2 *at 4 µM peptide* concentration.

A "selective inhibition" comprises an at least 20 % inhibition compared to a control and "significant inhibition" stands for an at least 40 % inhibition compared to a control without inhibitor.

For screening of the phage display libraries for both PSA- and hK2- binding peptides, the target enzyme was captured with a monoclonal antibody (MAb), which presents the active site of these enzymes efficiently to the potential ligands. We have previously identified specific PSA-binding peptides by using the same methodology as applied in the present invention (Wu, Leinonen et al. 2000), but contrary to the PSA-binding peptides, which enhanced the enzyme activity of PSA, the present peptides are hK2-inhibiting.

The term "amino acid" as used herein means an organic compound containing both a basic amino group and an acidic carboxyl group. Included within this term are natural amino acids (e.g., L-amino acids), modified and unnatural amino acids (e.g. (β-alanine), as well as amino acids which are known to occur biologically in free or combined form but usually do not occur in proteins. Included within this term are modified and unusual amino acids, such as those disclosed in, for example, Roberts and Vellaccio, 1983, the teaching of which is hereby incorporated by reference. Genetically coded, "natural" amino acids occurring in proteins include, but are not limited to, alanine, arginine, asparagine, aspartic acid, cysteine, glutamic acid, glutamine, glycine, histidine, isoleucine, leucine, lysine, methionine, phenylalanine, serine, threonine, tyrosine, tryptophan, proline, and valine. Natural non-protein amino acids include, but are not limited to arginosuccinic acid, citrulline, cysteine sulfinic acid, 3,4-dihydroxyphenylalanine, homocysteine, homoserine, omithine, 3-monoiodotyrosine, 3,5-diiodotryosine, 3,5,5'-triiodothyronine, and 3,3',5,5'-tetraiodothyronine. Modified or unusual amino acids which can be used to practice the invention include, but are not limited to, D-amino acids, hydroxylysine, 4-hydroxyproline, an N-Cbz-protected amino acid, 2,4-diaminobutyric acid, homoarginine, norleucine, N-methylaminobutyric acid, naphthylalanine, phenylglycine, 9-phenylproline, tert-leucine, 4-aminocyclohexylalanine, N-methyl-norleucine, 3,4-dehydroproline, N,N-dimethyl-aminoglycine, N-methylaminoglycine, 4-aminopiperidine-4-carboxylic acid, 6-aminocaproic acid, trans-4-(aminomethyl)-cyclohexanecarboxylic acid, 2-, 3-, and 4-(aminomethyl)-benzoic acid, 1-aminocyclopentanecarboxylic acid, 1-aminocyclopropane-carboxylic acid, and 2-benzyl-5-aminopentanoic acid.

Generally, "peptide" stands for a strand of several amino acids bonded together by amide bonds to form a peptide backbone. The term "peptide", as used herein, includes compounds containing both peptide and non-peptide components, such as pseudopeptide or peptidomimetic residues or other non-amino acid components. Such a compound containing both peptide and non-peptide components may also be referred to as a "peptide analog".

"Peptidomimetic compounds" are compounds, which resemble the original peptides mentioned above. They are generally built up of different chemical building blocks than the amino acids, which form the original peptides. For example, non-peptidyl compounds like benzolactam or piperazine based analogues based on the primary sequence of the original peptides can be used (Nargund et al. 1998, Houghten et al. 1999).

The resemblance between the peptidomimetic compounds and the original peptides is based on structural and functional similarities. Thus, the peptidomimetic compounds mimic the bioactive conformation of the original peptides and, for the purpose of the present application, their binding activity with respect to the binding site of hK2 is similar to that of the peptide they resemble. The peptidomimetic compounds can be made up of amino acids (such as D-amino acids), which do not appear in the original peptides, or they can be made from other compounds forming amide bonds or even ester bonds. Examples of synthetic peptidomimetic compounds comprise poly(ester imide)s, polyesters, N-alkylamino cyclic urea, thiourea, bicyclic guanidines, imidazol-pyridino-indoles, hydantoins and thiohydantoins (Nargund et al. 1998, Houghten et al. 1999). They may contain, e.g., the following groups: phenyl, cyclopentyl, cyclopentanyl, cyclohexenyl, cyclohexanyl, naphthyl, indanyl, furyl, thienyl, pyrrolyl, pyrrolinyl, pyrrolidinyl, pyrazolyl, pyrazolinyl, pyrazolidinyl, pyridyl, imidazoyl, imidazolinyl, imidazolidinyl, morpholinyl, piperidinyl, pyrazinyl, piperazinyl, pyrimidinyl, pyridazinyl, oxazolyl, oxazolidinyl, isoxazolyl, isoxazolidinyl, isothiazolidinyl, thiazolyl, thiazolidinyl, isothiazolyl, and bicyclic rings.

The peptidomimetic compounds can be characterized as being "functionally equivalent" to the peptides. The design of peptidomimetic compounds will be discussed in more detail below.

"Peptidyl analogues" or "conservative substitution-analogs" are chemical derivatives of the peptides based on the modification of the peptides by various chemical reactions, such as cycloadditions, condensation reactions and nucleophilic additions.

The present peptides comprise molecules with a molecular weight lower than 10 kDa, i.e. containing about 90 amino acids or less. Peptides can be designated as "small peptides" when they consist of about 6 ― 30 amino acid units. As mentioned above, the present peptides are generally linear. However, they may also comprise one or more cross-links formed by disulfide bonding between cysteine units. If the peptides contain several pairs of cysteines, there can be a multiple number of such cross-links. In addition to disulfide bonds, there can be other cross-links within the peptides as well.

The specific structures of some exemplary peptides are discussed in more detail below.

According to a specific embodiment of the invention, the novel binding and potentially also inhibiting agent for hK2 is
a. a peptide having at least 6 amino acids bonded together to form a peptide backbone and including an amino acid which is recognizable by the hK2 as an arginine unit or another basic side chain amino acid unit (e.g. lysine), or
b. a peptidomimetic compound having a spatial conformation similar to the peptide (mimicing the bioactive conformation of the original peptide).

Both of said compounds exhibit selective binding to hK2 and are capable of inhibiting, in particularly of specifically inhibiting, the proteolytic activity thereof.

According to a general definition of the present invention, the novel hK2 binding peptide ligands are based on structures according to formula (I)

X¹ X² X³ X⁴,

wherein
- X¹: is an optional residue of an amino acid selected from the group of R, K, F, Y, S, A and conservative substitutes thereof;
- X²: stands for a residue of an amino acid selected from the group of R, K and A and conservative substitutes thereof;
- X³: is a residue of an amino acid selected from the group of F, P, G, Q, M, R, K and conservative substitutes thereof; and
- X⁴: is a residue of an amino acid selected from the group of R, K, F, G, P and conservative substitutes thereof.

In particular, as will appear from an examination of the various meanings of the amino acid residues further below, the isolated peptides can also be represented by Formulas II

X¹¹ R R P X¹² P II

or III

X¹³ R F K X¹⁴ W III

wherein each X¹¹, X¹², X¹³ and X¹⁴ stands independently for the residue of a natural amino acid.

Further, in formulas II and III, X¹¹ and X¹³ are independently selected preferably from A or S and conservative substitutes thereof. Preferably, residues X¹² and X¹⁴ stand independently of the meaning of the other amino acid residues for A, F or V or conservative substitutes thereof. Thus, the present isolated peptides can also be presented by Formula IV

X²¹ X²² X²³ X²⁴ X²⁵ X²⁶ IV

wherein
- X²¹: is a residue of A, S or a conservative substitute thereof;
- X²²: is a residue of R or a conservative substitute thereof;
- X²³: is a residue of an amino acid selected from the group of R, F and conservative substitutes thereof;
- X²⁴: is a residue of an amino acid selected from the group of P, K and conservative substitutes thereof;
- X²⁵: is a residue of an amino acid selected from the group of A, F, V and conservative substitutes thereof; and
- X²⁶: is a residue of an amino acid selected from the group of P, W and conservative substitutes thereof.

A common factor for all of the peptides originally isolated by phage display was the presence of one or two Arg-residues. hK2 is known to cleave substrates after Arg-residues and all of the peptides identified contain Arg-residues. The presence of Arg-residues in all peptides identified suggests that they are important mediators of the binding to hK2. The Arg residues can, however, be replaced by residues having a similar chemical and physical structure and function, i.e. by basic amino acid residues, which are "identifiable" by hK2 as explained above.

Amino acid substitution and deletion analysis further showed that the motifs required for inhibitory activity of the peptides include motifs based on two basic structures, viz. RFKXW (SEQ ID NO:96) and ARRPXP (SEQ ID NO:94)(each of F, K and W, and A, R and P potentially being replaced by conservative substitutes). The common feature of peptides of motif RFKVWW representing SEQ ID NO:96, is a high pI, which can be calculated as being 11.00. The calculated pl of peptides of motif RRPAP representing SEQ ID NO:94 is even higher, namely 12.00.

A particularly strong inhibition is exhibited by the motif ARFKVWWAAG (SEQ ID NO:56).

Because the peptides contain R, and in some cases K residues, which ― as stated above ― are potential cleavage sites for trypsin, the peptides were analyzed for potential cleavage by trypsin. However, no cleavage was observed. Thus, the present peptides are unique in the respect that they are specific peptide inhibitors of hK2. By contrast, specific peptide substrates have been developed previously for hK2 by substrate phage display (Cloutier, Chagas et al. 2002) and on the basis of these, engineered hK2 specific serine protease inhibitors have been developed (Cloutier et al. 2004).

Comparison of the sequences of the substrate peptides with those of the present inhibitory peptides does not show significant sequence similarity, except that Arg was present in all peptides isolated in both studies.

The peptides were synthesized chemically and their effect on hK2 enzyme activity were evaluated by using a peptide substrate for human kallikreins. The peptides inhibited hK2 activity in a dose-dependent manner.

The peptides did not show any significant effect on the activity of PSA, α-chymotrypsin, bovine trypsin, human trypsin 1-3, plasmin, thrombin, urokinase and plasma kallikreinshowing that the peptides act specifically on hK2. In peptides selected from non-constrained libraries the loops have 4 - 5 amino acids between the Cys residues. Thus, it is possible that a cyclic peptide reacting with the active site of hK2 requires a small loop with a size of 4 - 5 amino acid residues to fit into the groove containing the catalytic residues.

It can be deduced that for specific inhibition of hK2 to occur, particularly preferred motifs comprise the amino acid sequences RFKXW (SEQ ID NO:96) or more preferred motif RFKVW (SEQ ID NO:97) or even more preferred motif ARFKVWW (SEQ ID NO:98), or RRPXP (SEQ ID NO:93) or more preferred motif ARRPXP (SEQ ID NO:94) or even more preferred motif ARRPFP (SEQ ID NO:95).

Inhibition can further be strengthened by incorporating spacer residues around the motifs. Thus, according to one preferred embodiment, strong inhibition is provided by derivatives of ARFKVWW motif (SEQ ID NO:98). Thus, according to Lineweaver-Burke Plot, the results of which are accounted for below, the peptide ARFKVWWAAG (SEQ ID NO:56) is a competitive inhibitor of hK2.

Based on the above, the following preferred embodiments of peptides according to Formula I have been found:
- Peptides having Formula I, comprising an amino acid sequence consisting of a minimum of 6 amino acid residues.
- Peptides having Formula I, further comprising an amino acid sequence having the formula X⁵, linked to the carboxy terminus of X⁴, wherein X⁵ comprises I to 6 amino acid residues selected from the group of natural and synthetic amino acids.
- Peptides having Formula I, further comprising an amino acid sequence having the formula X⁻¹, linked to the amino terminus of X¹, wherein X₋₁ comprises 1 to 4 amino acids selected from the group of natural and synthetic amino acids.
- Peptides having Formula I, further comprising cysteine residues, the cysteine residues in particular residing at the carboxy and amino termini.
- Peptides having Formula I, wherein X⁵ is an amino acid residue selected from P, A, Sar, V, C and T and conservative substitution-analogs thereof.
- Peptides having Formula I, further comprising an amino acid residue X⁶ linked to the carboxy terminus of X₅, wherein X⁶ is a residue derived from an amino acid selected from the group of P, A, W, G, C and V and conservative substitution-analogs thereof.
- Peptides having Formula I, further comprising an amino acid residue X⁷ linked to the carboxy terminus of X⁶, wherein X⁷ is a residue derived from an amino acid selected from the group of P,A,T,W,G,M,V and C and conservative substitution-analogs thereof.
- Peptides having Formula I, further comprising an amino acid residue X⁸ linked to the carboxy terminus of X⁷, wherein X⁸ is a residue derived from an amino acid selected from the group of S, A, G, Q, I and C and conservative substitution-analogs thereof.
- Peptides having Formula I, further comprising a residue X⁹ linked to the carboxy terminus of X⁸, wherein X⁹ is a residue derived from G, A, C, (CONH₂) and conservative substitution-analogs thereof.
- Peptides having Formula I, further comprising a residue X¹⁰ linked to the carboxy terminus of X⁹, wherein X¹⁰ is a residue derived from G, D, C, (CONH₂) and conservative substitution-analogs thereof, and
- Peptides having Formula I, wherein X⁻¹ is a residue derived from an amino acid selected from A, G and C and conservative substitution-analogs thereof and optionally comprising an amino acid residue X⁻² linked to the amino terminus of X⁻¹, wherein X⁻² is a residue derived from A, G, P and C and conservative substitution-analogs thereof. These peptides may also comprise an amino acid residue X⁻³ linked to the amino terminus of X⁻², wherein X⁻³ is a residue derived from A, G and C and conservative substitution-analogs thereof.

The above peptides provide excellent binding and inhibition properties. Each of the above, preferred embodiments may comprise about residues of 6 to 90 amino acids, preferably about 6 to 50 amino acids, in particular about 6 to 30 amino acids, in the peptide backbone.

In the above peptides, X⁵ is preferably selected from the group consisting of PAPS (SEQ ID NO:99), AAPS (SEQ ID NO: 100), PAAS (SEQ ID NO:101), PAPA (SEQ ID NO:102), PVTA (SEQ ID NO:103), PATA (SEQ ID NO:104), PVAA (SEQ ID NO:105), PVT (SEQ ID NO:106), PV (SEQ ID NO: 107), PA (SEQ ID NO: 108), PAP (SEQ ID NO:109), SarAP (SEQ ID NO:110), VWWAA (SEQ ID NO:111), AWWAA (SEQ ID NO:112), VAWAA (SEQ ID NO: 113), VWAAA (SEQ ID NO: 114), VWWA (SEQ ID NO: 115), VWW (SEQ ID NO: 116), VW (SEQ ID NO: 117), VG (SEQ ID NO:118), VWWG (SEQ ID NO: 119), and VWWAA (SEQ ID NO:120) and permutations thereof.

X⁻¹ is, also preferably, selected from the group consisting of GAA (SEQ ID NO:121), GPA (SEQ ID NO:122), GA (SEQ ID NO: 123), AA (SEQ ID NO:124), AAA (SEQ ID NO:125) and AAA (SEQ ID NO:126) and permutations thereof.

Generally, the novel isolated peptides are linear. Some cyclic peptides have also been found. As specific examples, the following can be particularly mentioned, although some others are also disclosed in Table 5:
G, C, A, A, R, F, K, V, W, W, A, A, C, G (SEQ ID NO: 84)
G, C, A, R, F, K, V, W, W, A, C, G (SEQ ID NO: 128)
G, C, R, F, K, V, W, W, C, G (SEQ ID NO: 129)

Where C3 stands for cystein (C) with or without an acetoamido methyl (Acm) protective group.

The terms "conservative substitution" and "conservative substitutes" as used herein denote the replacement of an amino acid residue by another, biologically similar residue with respect to hydrophobicity, hydrophilicity, cationic charge, anionic charge, shape, polarity and the like. Examples of conservative substitutions include the substitution of one hydrophobic residue such as isoleucine, valine, leucine, alanine, cysteine, glycine, phenylalanine, proline, tryptophan, tyrosine, norleucine or methionine for another, or the substitution of one polar residue for another, such as the substitution of arginine for lysine, glutamic acid for aspartic acid, or glutamine for asparagine, and the like. Neutral hydrophilic amino acids, which can be substituted for one another, include asparagine, glutamine, serine and threonine. The term "conservative substitution" also includes the use of a substituted or modified amino acid in place of an unsubstituted parent amino acid provided that substituted peptide reacts with hK2. By "substituted" or "modified" the present invention includes those amino acids that have been altered or modified from naturally occurring amino acids.

As such, it should be understood that in the context of the present invention, a conservative substitution is recognized in the art as a substitution of one amino acid for another amino acid that has similar properties. Exemplary conservative substitutions are set out in the Table I below:

**Table 1. Conservative Substitutions I**

| SIDE CHAIN CHARACTERISTIC | AMINO ACID |
|---|---|
| Non-polar | G A P I L V |
| Polar-uncharged | C S T M N Q |
| Polar-charged | D E K R |
| Aromatic | H F W Y |
| Other | N Q D E |

Alternatively, conservative amino acids can be grouped as described in Lehninger, 1975, as set out in Table 2, immediately below.

**Table 2. Conservative Substitutions II**

| SIDE CHAIN CHARACTERISTIC | AMINO ACID |
|---|---|
| Non-polar (hydrophobic) | |
| A. Aliphatic: | A L I V P |
| B. Aromatic: | F W |
| C. Sulfur-containing: | M |
| D. Borderline: | G |
| | |
| Uncharged-polar | |
| A. Hydroxyl: | S T Y |
| B. Amides: | N Q |
| C. Sulfhydryl: | C |
| D. Borderline: | G |
| Positively Charged (Basic): | K R H |
| Negatively Charged (Acidic): | D E |

As still another alternative, exemplary conservative substitutions are set out in Table 3, immediately below.

**Table 3. Conservative Substitutions III**

| Original Residue | Exemplary Substitution |
|---|---|
| Ala (A) | Val (V), Leu (L), lle (I) |
| Arg (R) | Lys (K), Gln (Q), Asn (N) |
| Asn (N) | Gln (Q), His (H), Lys (K), Arg (R) |
| Asp (D) | Glu (E) |
| Cys (C) | Ser (S) |
| Gln (Q) | Asn (N) |
| Glu (E) | Asp (D) |
| His (H) | Asn (N), Gln (Q), Lys (K), Arg (R) |
| Ile (I) | Leu (L), Val (V), Met (M), Ala (A), Phe (F) |
| Leu (L) | Ile (I), Val (V), Met (M), Ala (A), Phe (F) |
| Lys (K) | Arg (R), Gln (Q), Asn (N) |
| Met (M) | Leu (L), Phe (F), Ile (I) |
| Phe (F) | Leu (L), Val (V), lle (I), Ala (A) |
| Pro (P) | Gly (G) |
| Ser (S) | Thr (T) |
| Thr (T) | Ser (S) |
| Trp (W) | Tyr (T) |
| Tyr (Y) | Trp (W), Phe (F), Thr (T), Ser (S) |
| Val (V) | lle (I), Leu (L), Met (M), Phe (F), Ala (A) |

Any conservative variant of X¹ X² X³ X⁴ (Formula I) is contemplated to be a useful peptide of the present invention as long as such a variant retains its property of binding to hK2. Additionally it is contemplated that non-conservative variants of these peptides also may be designed that may prove to be more efficient inhibitors of hK2 enzyme activity/binders than the original X¹ X² X³ X⁴ (Formula I) identified by the present invention.

Based on the above, five groups of preferred peptides can be listed, viz.:
Isolated peptides comprising an amino acid sequence selected from the group consisting of: GAARRPFPAPSG (SEQ ID NO:7), GAARRPAPAPSG (SEQ ID NO: 11), GAARRPFAAPSG (SEQ ID NO:12), GAARRPFPAASG (SEQ ID NO:13), GAARRPFPAPAG (SEQ ID NO:14), GPARRPFPVTAG (SEQ ID NO:15), GAARRPFPVTAG, (SEQ ID NO:16), GPARRPAPVTAG (SEQ ID NO:20), GPARRPFAVTAG (SEQ ID NO:21), GPARRPFPATAG (SEQ ID NO:22) and GPARRPFPVAAG (SEQ ID NO:23).
Isolated peptides comprising an amino acid sequence selected from the group consisting of: GPARRPFPVTG (SEQ ID NO:24), GPARRPFPVG (SEQ ID NO:25), GPARRPFPG (SEQ ID NO:26), GPARRPFPVTAG (SEQ ID NO:31), GARRPFPVTAG (SEQ ID NO:32), AARRPAPG (SEQ ID NO:45), AAARRPAPG (SEQ ID NO:46), AARRPFPG (SEQ ID NO:47), AAARRPFPG (SEQ ID NO:48), AAARRPAPA (SEQ ID NO:49), AAARRPAPG (SEQ ID NO:50), GAARRPFPAPG (SEQ ID NO:51) and GAARRPFSarAPG (SEQ ID NO:53).
Isolated peptides comprising an amino acid sequence selected from the group consisting of: SRFKVWWAAG (SEQ ID NO:55), ARFKVWWAAG (SEQ ID NO:56), SAFKVWWAAG (SEQ ID NO:57), SRFKAWWAAG (SEQ ID NO:60), SRFKVAWAAG (SEQ ID NO:61), SRFKVWAAAG (SEQ ID NO:62), SRFKVWWAAG (SEQ ID NO:63), SRFKVWWAAG (SEQ ID NO:64), RFKVWWAAG (SEQ ID NO:65), SRFKVWWAG (SEQ ID NO:68), SRFKVWWG (SEQ ID NO:69).
Isolated peptides comprising an amino acid sequence selected from the group consisting of: SRFKVWWG (SEQ ID NO:73), SRFKVWWG (SEQ ID NO:74), ARFKVWWG (SEQ ID NO:75), ARFKVWWG (SEQ ID NO:76), ARFKVWWGG (SEQ ID NO:77), ARFKVWWGG (SEQ ID NO:78), ARFKVWWA (SEQ ID NO:79), ARFKVWWA (SEQ ID NO:80), ARFKVWWAA (SEQ ID NO:81) and ARFKVWWA(CONH₂) (SEQ ID NO:82).
Isolated peptides comprising an amino acid sequence selected from the group consisting of: SRFKVWWAAG (SEQ ID NO:1), AARRPFPAPS (SEQ ID NO:2), PARRPFPVTA (SEQ ID NO:3), CFRQGCWVIT (SEQ ID NO:4) and CYRMPTCMQRD (SEQ ID NO:6).

The preferred peptides of the present invention can be made stable and protease resistant by preparing cyclic peptides (SEQ ID NO:84), wherein the cysteine residues are replaced by amino acids making a new bond. Such a library of backbone cyclic peptides may be prepared in E. coli using suitable expression systems, such as, but not limited to, an intein-based expression system. Methods of screening these peptides for higher binding affinity are described elsewhere in the specification.

Other methods of constructing peptides which are resistant to proteolytic digestion are also possible, such as peptides including non-hydrolyzable peptide bonds, and peptides having end modifications such as an amide (e.g., CONH₂) at the C-terminus or a acetyl group at the N-terminus. It is contemplated that the peptides of the invention are modified such that their *in vivo* half life is increased, their physical stability is increased, rate of *in vivo* release and rate of *in vivo* clearance also may be affected.

Preferably, the inhibitory peptides of the present invention are non-hydrolyzable. To provide such peptides, one may select peptides from a library of non-hydrolyzable peptides, such as peptides containing one or more D-amino acids or peptides containing one or more non-hydrolyzable peptide bonds linking amino acids. Alternatively, one can select peptides, which are optimal inhibitors of hK2, and then modify such peptides as necessary to reduce the potential for hydrolysis by other proteases. For example, to determine the susceptibility to proteolytic cleavage, peptides may be labeled and incubated with cell extracts, plasma or purified proteases and then isolated to determine which peptide bonds are susceptible to proteolysis, e.g., by mass spectrometry and sequencing of peptides and proteolytic fragments. Alternatively, potentially susceptible peptide bonds can be identified by comparing the amino acid sequence of the inhibitory peptides of the present invention with the known cleavage site specificity of a panel of proteases. Based on the results of such assays, individual peptide bonds, which are susceptible to proteolysis, can be replaced by non-hydrolyzable peptide bonds by in vitro synthesis of the peptide.

Many non-hydrolyzable peptide bonds are known in the art, along with procedures for synthesis of peptides containing such bonds. Non-hydrolyzable bonds include --[CH₂NH]-reduced amide peptide bonds, --[COCH₂]-- ketomethylene peptide bonds, --[CH(CN)NH]-- (cyanomethylene)amino peptide bonds, --[CH₂CH(OH)]-- hydroxyethylene peptide bonds, --[CH₂O]-- peptide bonds, and --[CH₂S]-- thiomethylene peptide bonds (see e.g., U.S. Pat. No. 6,172,043).

Peptides useful in the invention can be linear, or maybe circular or cyclized by natural or synthetic means. For example, disulfide bonds between cysteine residues may cyclize a peptide sequence. Bifunctional reagents can be used to provide a linkage between two or more amino acids of a peptide. Other methods for cyclization of peptides, such as those described by Anwer et al. (1990) and Rivera-Baeza et al. (1996), are also known in the art.

Furthermore, non-peptide analogs of peptides, which provide a stabilized structure or lessened biodegradation, are also contemplated. Peptide mimetic analogs can be prepared based on a selected inhibitory peptide by replacement of one or more residues by non-peptide moieties. Preferably, the non-peptide moieties permit the peptide to retain its natural conformation, or stabilize a preferred, e.g., bioactive, conformation. One example of methods for preparation of non-peptide mimetic analogs from peptides is described in Nachman et al. (1995). The term "peptide" as used herein embraces all of the foregoing.

If desired, the peptides of the invention can be modified, for instance, by glycosylation, amidation, carboxylation, pegylation, or phosphorylation, or by the creation of acid addition salts, amides, esters, in particular C-terminal esters, and N-acyl derivatives of the peptides of the invention. The peptides also can be modified to create peptide derivatives by forming covalent or non-covalent complexes with other moieties. Covalently bound complexes can be prepared by linking the chemical moieties to functional groups on the side chains of amino acids comprising the peptides, or at the N- or C-terminus

In particular, it is anticipated that the aforementioned peptides can be conjugated to a reporter group, including, but not limited to a radiolabel, a fluorescent label, an enzyme (e.g., that catalyzes a colorimetric or fluorometric reaction), a substrate, a solid matrix, or a carrier (e.g., biotin or avidin). The invention accordingly provides a molecule comprising a peptide inhibitor of hK2, wherein the molecule preferably further comprises a reporter group selected from the group consisting of a radiolabel, a fluorescent label, an enzyme, a substrate, a solid matrix, and a carrier. Such labels are well known to those of skill in the art, e.g., biotin labels are particularly contemplated. The use of such labels is well known to those of skill in the art and is described in, e.g., U.S. Pat. Nos. 3,817,837; 3,850,752; 3,996,345 and 4,277,437. Other labels that will be useful include, but are not limited to, radioactive labels, fluorescent labels and chemiluminescent labels. U.S. Patents concerning use of such labels include for example U.S. Pat. Nos. 3,817,837; 3,850,752; 3,939,350 and 3,996,345. Any of the peptides of the present invention may comprise one, two, or more of any of these labels.

The binding and inhibiting peptides of the invention will be used as therapeutic compositions either alone or in combination with other therapeutic agents. For such therapeutic uses small molecules are generally preferred because the reduced size renders such peptides more accessible for uptake by the target. It is contemplated that the preferred peptides of the present invention are from about 6, 7, 8, 9, or 10 amino acid residues in length to about 90 or 100 amino acid residues in length. Of course it is contemplated that longer or indeed shorter peptides also may prove useful. Thus, peptides of 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95 and a 100 amino acids in length will be particularly useful. Such peptides may be present as individual peptides or may coalesce into dimers or multimers for greater efficacy.

The present invention also involves, in another embodiment, the treatment of pathologies characterized by hK2 activity. A disorder that may benefit from an intervention using the peptides of the present invention includes, but are not limited to prostate neoplasm, prostate cancer, metastatic prostate cancer and the like. The peptides of the present invention will be useful against any disorder that is mediated through the proteolytic activity of hK2. It is contemplated that these peptides also will have a tumor spread inhibiting effect in a prostate cancer.

In many contexts, it is not necessary that the tumor cell be killed or induced to undergo cell death. Rather, to accomplish a meaningful treatment, all that is required is that the tumor growth be slowed to some degree or localized to a specific area and inhibited from spread to distant sites. It may be that the tumor growth is completely blocked, however, or that some tumor regression is achieved. Clinical terminology such as "remission" and "reduction of tumor" burden also are contemplated given their normal usage. In the context of the present invention, the therapeutic effect may result from an inhibition of angiogenesis.

The peptides of the present invention will be used for the therapeutic intervention of the disorders discussed herein above as well as any other disorders that are mediated through the proteolytic activity of hK2. These therapies will be particularly useful as anti-metastatic and/or anti-angiogenic treatments, however it is contemplated that the instant invention is not limited to these beneficial effects. By "mediated through" the present invention refers to any biological events that result from the proteolytic activity of hK2, such as, for example, result from hK2 mediated growth or that result from an aberration in the expression or activity of hK2. As discussed above, the peptides may be produced by recombinant expression means or generated by an automated peptide synthesizer. Formulations would be selected based on the route of administration and purpose including, but not limited to, liposomal formulations and classic pharmaceutical preparations.

Administration of the compositions can be systemic or local and may comprise a single site injection of a therapeutically effective amount of the peptide composition of the present invention. Any route known to those of skill in the art for the administration of a therapeutic composition of the invention is contemplated including for example, intravenous, intramuscular, subcutaneous or a catheter for long-term administration. Alternatively, it is contemplated that the therapeutic composition may be delivered to the patient at multiple sites. The multiple administrations may be rendered simultaneously or may be administered over a period of several hours. In certain cases it may be beneficial to provide a continuous flow of the therapeutic composition. Additional therapy may be administered on a period basis, for example, daily, weekly or monthly.

In general, per oral dosage forms for the therapeutic delivery of peptides is ineffective because in order for such a formulation to the efficacious, the peptide must be protected from the enzymatic environment of the gastrointestinal tract. Additionally, the peptide must be formulated such that it is readily absorbed by the intestinal epithelial cell barrier in sufficient concentrations to effect a therapeutic outcome. The peptides of the present invention may be formulated with uptake- or absorption-enhancers to increase their efficacy. Such enhancer include for example, salicylate, glycocholate/linoleate, glycholate, aprotinin, bacitracin, SDS caprate and the like. Also drugs developed for treatment of HIV, based on inhibition of the protease of HIV are included.

The amounts of peptides in a given dosage will vary according to the size of the individual to whom the therapy is being administered as well as the characteristics of the disorder being treated. In exemplary treatments, it may be necessary to administer about 50 mg/day, 75 mg/day, 100 mg/day, 150 mg/day, 200 mg/day or 250 mg/day. These concentrations may be administered as a single dosage form or as multiple doses.

The peptides of the present invention also may be provided by using nucleic acid constructs. Specifically, cancer or other cells may be contacted with an expression construct capable of expressing the peptides of the present invention in a manner to allow the inhibition of hK2. However, it seems that it is more likely that the therapeutic aspects of the present invention may employ gene-based therapies in combination with the peptide therapies of the present invention as discussed herein below.

For these embodiments, an exemplary expression construct comprises a virus or engineered construct derived from a viral genome. The expression construct generally comprises a nucleic acid encoding the gene to be expressed and also additional regulatory regions that will effect the expression of the gene in the cell to which it is administered. Such regulatory regions include for example promoters, enhancers, polyadenylation signals and the like.

It is now widely recognized that DNA may be introduced into a cell using a variety of viral vectors. In such embodiments, expression constructs comprising viral vectors containing the genes of interest may be adenoviral (see for example, U.S. Pat. Nos. 5,824,544; 5,707,618; 5,693,509; 5,670,488; 5,585,362; each incorporated herein by reference), retroviral (see for example, U.S. Pat. Nos. 5,888,502; 5,830,725; 5,770,414; 5,686,278; 4,861,719 each incorporated herein by reference), adeno-associated viral (see for example, U.S. Pat. Nos. 5,474,935; 5,139,941; 5,622,856; 5,658,776; 5,773,289; 5,789,390; 5,834,441; 5,863,541; 5,851,521; 5,252,479 each incorporated herein by reference), an adenoviral-adenoassociated viral hybrid (see for example, U.S. Pat. No. 5,856,152 incorporated herein by reference) or a vaccinia viral or a herpesviral (see for example, U.S. Pat. Nos. 5,879,934; 5,849,571; 5,830,727; 5,661,033; 5,328,688 each incorporated herein by reference) vector.

In other embodiments, non-viral delivery is contemplated. These include calcium phosphate precipitation (Rippe et al., 1990) DEAE-dextran (Gopal, 1985), electroporation (Tur-Kaspa et al., 1986), direct microinjection (Harland and Weintraub, 1985), DNA-loaded liposomes (Felgner, 1997), cell sonication (Fechheimer et al., 1987), gene bombardment using high velocity microprojectiles (Yang et al., 1990), and receptor-mediated transfection (Wu and Wu, 1993).

In a particular embodiment of the invention, the expression construct (or indeed the peptides discussed above) may be entrapped in a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers. The addition of DNA to cationic liposomes causes a topological transition from liposomes to optically birefringent liquid-crystalline condensed globules. These DNA-lipid complexes are potential non-viral systems for use in gene therapy and delivery.

Liposome-mediated nucleic acid delivery and expression of foreign DNA in vitro has been very successful. Also contemplated in the present invention are various commercial approaches involving "lipofection" technology. In certain embodiments of the invention, the liposome may be complexed with a hemagglutinating virus (HVJ). This has been shown to facilitate fusion with the cell membrane and promote cell entry of liposome- encapsulated DNA. In other embodiments, the liposome may be complexed or employed in conjunction with both HVJ and HMG-1. In that such expression constructs have been successfully employed in transfer and expression of nucleic acid in vitro and in vivo, then they are applicable for the present invention.

Other vector delivery systems that can be employed to deliver a nucleic acid encoding a therapeutic gene into cells include receptor-mediated delivery vehicles. These take advantage of the selective uptake of macromolecules by receptor-mediated endocytosis in almost all eukaryotic cells. Because of the cell type-specific distribution of various receptors, the delivery can be highly specific.

In other embodiments, the delivery vehicle may comprise a ligand and a liposome. For example, Nicoiau with coworkers (Nicoiau et al. 1987) employed lactosyl-ceramide, a galactose-terminal asialganglioside, incorporated into liposomes and observed an increase in the uptake of the insulin gene by hepatocytes. Thus, it is feasible that a nucleic acid encoding a therapeutic gene also may be specifically delivered into a particular cell type by any number of receptor-ligand systems with or without liposomes.

In another embodiment of the invention, the expression construct may simply consist of naked recombinant DNA or plasmids. Transfer of the construct may be performed by any of the methods mentioned above that physically or chemically permeabilize the cell membrane. This is applicable particularly for transfer in vitro, however, it may be applied for in vivo use as well.

Another embodiment of the invention for transferring a naked DNA expression construct into cells may involve particle bombardment. This method depends on the ability to accelerate DNA coated microprojectiles to a high velocity allowing them to pierce cell membranes and enter cells without killing them. Several devices for accelerating small particles have been developed. One such device relies on a high voltage discharge to generate an electrical current, which in turn provides the motive force (Yang et al., Proc. Natl. Acad. Sci USA, 87:9568-9572, 1990). The microprojectiles used have consisted of biologically inert substances such as tungsten or gold beads.

Those of skill in the art are well aware of how to apply gene delivery to in vivo and ex vivo situations. For viral vectors, one generally will prepare a viral vector stock. Depending on the kind of virus and the titer attainable, one will deliver 1x10⁴, 1x10⁵, 1x10⁶, 1x10⁷, 1x10⁸, 1x10⁹, 1x10¹⁰, 1x10¹¹ or 1x10¹² infectious particles to the patient. Similar figures may be extrapolated for liposomal or other non-viral formulations by comparing relative uptake efficiencies. Formulation as a pharmaceutically acceptable composition is discussed below.

Various routes are contemplated for various tumor types. The section below contains an extensive list of possible routes. For practically any tumor, systemic delivery is contemplated. This will prove especially important for attacking microscopic or metastatic cancer. Where discrete tumor mass may be identified, a variety of direct, local and regional approaches may be taken. For example, the tumor may be directly injected with the expression vector or protein. A tumor may be treated prior to, during or after resection. Following resection, one generally will deliver the vector by a catheter left in place following surgery. One may utilize the tumor vasculature to introduce the vector into the tumor by injecting a supporting vein or artery. A more distal blood supply route also may be utilized.

In a different embodiment, *ex vivo* gene therapy is contemplated. In an *ex vivo* embodiment, cells from the patient are removed and maintained outside the body for at least some period of time. During this period, a therapy is delivered, after which the cells are reintroduced into the patient; hopefully, any tumor cells in the sample have been killed.

The present invention also relates to a pharmaceutical composition comprising an amount of the novel hK2 inhibitors. The pharmaceutical composition comprising the novel hK2 inhibiting peptides according to the invention may be used systemically, locally and/or topically, and may be administered e.g. parenterally, intravenously, subcutaneously, intramuscularly, intranasally, by pulmonary aerosol or in depot form. The compositions may also include all potential combinations of the peptides with labelling reagents, imaging reagents, drugs and other chemicals/-molecules.

Pharmaceutical compositions suitable for intravenous infusion or injection are particularly preferred and they comprise the active component in a concentration of, generally, about 0.1 to 500 g/l, preferably about 1 to 250 g/l. It is preferred to have somewhat higher concentrations (e.g. about 20 to 200 g/l), to allow for administration without causing excessive volume load to the patients. The preparation may be lyophilized and reconstituted before administration or it may be stored as a solution ready for administration. The pH of the solution product is in the range of about 5 to about 8, preferably close to physiological pH. The osmolality of the solution can be adjusted to a preferred value of at least 200 mOsmol/l (onko tämä litraa vai kiloa kohden?) using sodium chloride and/or sugars, polyols or amino acids or similar components. The compositions can further contain pharmaceutically acceptable excipients and stabilizers, such as albumin, sugars and various polyols. The amounts of these components can vary broadly within a range of about 0 to 50 wt-% of the active component. Liquid formulations provide the additional advantage of being ready for administration without reconstitution.

For oral administration it may be necessary to prepare derivatives of the present peptides.

According to a particularly interesting embodiment, the peptides (and peptidomimetics) are incorporated into a liposome package containing diagnostic/therapeutic compounds, such as cytotoxic drugs doxorubicin and methotrexate. Liposome packaging peptides are disclosed by Zalipsky et al. 1995 and Slepushkin et al. 1996. Coupling of the peptides to the surface of the liposome enables the targeting of it to hK2 producing cells.

The present invention also includes the use of the novel hK2 binding peptides for the manufacture of reagents for hK2 based diagnosis of benign and malignant prostatic disease and diseases derived from other tissues producing hK2.

The present invention also includes the use of novel hK2 binding peptides for the manufacture of the above-mentioned pharmaceutical preparations for the treatment and targeting of conditions based on the use of present invention. The present invention also includes the use of novel hK2 binding peptides for treatment of conditions based on regulation of hK2 enzyme activity. Thus, generally the novel peptides and structurally and functionally equivalent peptidoinimetics (= active components) can be employed in pharmaceutical compositions to treat mammalian cancers as well as other conditions, by administering an effective dose of the peptide or peptidomimetic or a therapeutically acceptable acid or salt or derivative thereof in a pharmaceutical carrier. They can be administered with a pharmaceutically acceptable carrier at dosages of from about 1 to about 1,000 micrograms per kg of body weight daily. As mentioned above the composition may be administered parenterally, intravenously, subcutaneously, intramuscularly, intranasally, by pulmonary aerosol or in depot form.

An additional use for the peptides of the present invention is in tissue imaging to detect prostate tissue or prostate cancer expressing hK2. The use of such diagnostic imaging is particularly suitable in obtaining an image of, for example, a tumor mass or the neovascularizarion near a tumor mass.

The peptides of the present invention may be coupled either covalently or noncovalently to a suitable supramagnetic, paramagnetic, electron-dense, echogenic or radioactive agent to produce a targeted imaging agent. In such embodiments, the peptide imaging agent will localize to the receptor and the area of localization be imaged using the above referenced techniques.

Many appropriate imaging agents are known in the art, as are methods of attaching the labeling agents to the peptides of the invention (see, e.g., U.S. Pat. Nos. 4,965,392, 4,472,509, 5,201,236 and 5,037,630, incorporated herein by reference). The labeled peptides are administered to a subject in a pharmaceutically acceptable carrier, and allowed to accumulate at a target site containing hK2. This peptide imaging agent then serves as a contrast reagent for X-ray, magnetic resonance, sonographic or scintigraphic imaging of the target site. The peptides of the present invention are a convenient and important addition to the available arsenal of medical imaging tools for the diagnostic investigation of cancer and especially prostate cancer.

Paramagnetic ions useful in the imaging agents of the present invention include for example chromium (III), manganese (II), iron (III), iron (II), cobalt (II), nickel (II) copper (II), neodymium (III), samarium (III), ytterbium (III), gadolinium (III), vanadium (II), terbium (III), dysprosium (III), holmium (III) and erbium (III). Ions useful for X-ray imaging include but are not limited to lanthanum (II), gold (III), lead (II) and particularly bismuth (III). Radioisotopes for diagnostic applications include for example, ²¹¹ astatine, ¹⁴carbon, ⁵¹chromium, ³⁶chlorine, ⁵⁷cobalt, ⁶⁷copper, ¹⁵²Eu, ⁶⁷gallium, ³hydrogen, ¹²³iodine, ¹²⁵iodine, ¹¹¹indium, ⁵⁹iron, ³²phosphorus, ¹⁸⁶rhenium, ⁷⁵selenium, ³⁵sulphur, ^{99m}technicium and ⁹⁰yttrium.

The peptides of the present invention may be labeled according to techniques well known to those of skill in the art. For example, the peptides can be iodinated by contacting the peptide with sodium or potassium iodide and a chemical oxidizing agent such as sodium hypochlorite or an enzymatic oxidant such as lactoperoxidase. Peptides may be labeled with ^{99m}technetium by ligand exchange, for example, by reducing pertechnate with stannous solution, chelating the reduced technetium onto a Sephadex column and applying the peptide to the column. Peptides may be coupled to different chleates, like DTPA or DOTA. Several chelate molecules may be coupled to one peptide in order to increase the sensitivity. Chleates may be labeled with technetium for imaging or indium for therapy. These and other techniques for labeling proteins and peptides are well known to those of skill in the art.

The present invention also relates to the use of the novel hK2 binding peptides for biochemical isolation and purification procedures of various forms of hK2.

The present invention also relates to a process for the preparation of these hK2 binding peptides by standard solid phase Merrifield peptide synthesis. More details on the synthesis of peptides and peptidomimetics are given below.

The peptides according to the present invention can be used as such or as labelled derivatives as part of quantitative assays for various molecular forms of hK2, or as compounds to inhibit the activity of hK2 or for targeting of hK2 producing cells.

The peptides can also be used in column chromatographic matrices for biochemical isolation and purification of various forms of hK2, as already discussed above.

Furthermore, as mentioned earlier, the present binding and inhibiting peptides can be used as lead compounds to design peptidomimetics for purposes described above. Such a method of deriving peptidomimetics of biologically active hK2 peptides can, for exampe, comprise the following steps:
(a) selecting a biologically active hK2 peptide,
(b) modeling a non-peptidic structure that is superimposable on the template space defined by the hK2 peptide, and
(c) forming a peptidomimetic by adding to the non-peptidic structure at least one element comprising an amino acid residue, amino acid side chain moiety or derivative thereof, such that at least an element occupies a similar descriptor space as the corresponding element of the biologically active hK2 peptide.

As discussed above, a "pseudopeptide" or "peptidomimetic" is a compound which mimics the structure of an amino acid residue or a peptide, for example, by using linking groups other than amide linkages between the peptide mimetic and an amino acid residue (pseudopeptide bonds) and/or by using non-amino acid substituents and/or a modified amino acid residue. A "pseudopeptide residue" means that portion of a pseudopeptide or peptidomimetic that is present in a peptide.

The present invention demonstrates that hK2 peptides generally can be used in the present invention, here peptides can also include polypeptides and other molecules, designated "mimetics" or "peptidomimetics", which have the capacity to interfere with hK2 enzyme function. Particularly preferred are compounds, which specifically interfere with hK2 function and do not interfere with function of other kallikreins or serine proteases.

The term "peptide bond" means a covalent amide linkage formed by loss of a molecule of water between the carboxyl group of one amino acid and the amino group of a second amino acid.

The term "pseudopeptide bonds" includes peptide bond isosteres, which may be used in place of or as substitutes for the normal amide linkage. These substitutes or amide "equivalent" linkages are formed from combinations of atoms not normally found in peptides or proteins which mimic the spatial requirements of the amide bond and which should stabilize the molecule to enzymatic degradation.

The term "non-peptide" refers to a compound, which is comprised of preferably less than three amide bonds in the backbone core compound or preferably less than three amino acids or amino acid mimetics.

The peptides, polypeptides, peptidomimetics and non-peptides, optionally bearing a linking group, or a fragment of the linking group, can be synthesized using standard synthetic methods known to those skilled in the art. Preferred methods include but are not limited to those methods described below.

As described previously, hK2 peptides can inhibit enzymatic activity of hK2 enzyme/polypeptide. The ability to inhibit the enzymatic activity of hK2 is desirable for compounds of the invention. In some embodiments, a compound of the invention may have structural characteristics that permit it to inhibit hK2 enzyme activity. In other embodiments, a compound of the invention may be linked to a carrier

Generally, peptides, polypeptides, and peptidomimetics are elongated by deprotecting the alpha-amine of the C-terminal residue and coupling the next suitably protected amino acid through a peptide linkage using the methods described. This deprotection and coupling procedure is repeated until the desired sequence is obtained. This coupling can be performed with the constituent amino acids in a stepwise fashion, or condensation of fragments (two to several amino acids), or combination of both processes, or by solid phase peptide synthesis according to the method originally described by Merrifield (Merrifield 1963), the disclosure of which is hereby incorporated by reference.

The peptides, polypeptides and peptidomimetics may also be synthesized using automated synthesizing equipment. In addition to the foregoing, procedures for peptide, polypeptide and peptidomimetic synthesis are described in Stewart and Young (1984), Gross, Meienhofer, Udenfriend (1980-1987), Bodanszky (1988), and Bodanszky et al. (1984), the disclosures of which are hereby incorporated by reference.

The coupling between two amino acid derivatives, an amino acid and a peptide, polypeptide or peptidomimetic, two peptide, polypeptide or peptidomimetic fragments, or the cyclization of a peptide, polypeptide or peptidomimetic can be carried out using standard coupling procedures such as the azide method, mixed carbonic acid anhydride (isobutyl chloroformate) method, carbodiimide (dicyclohexylcarbodiimide, diisopropylcarbodiimide, or water-soluble carbodiimides) method, active ester (p-nitrophenyl ester, N-hydroxysuccinic imido ester) method, Woodward reagent K method, carbonyldiimidazole method, phosphorus reagents such as BOP (Benzotriatzole-l-yl-oxy(dimethylamino)-phosphoriumhexafluorophosphate, Castro's reagent) and its uranium derivates HBTU and TBTU, or oxidation-reduction method. Some of these methods (especially the carbodiimide) can be enhanced by the addition of 1-hydroxybenzotriazole. These coupling reactions may be performed in either solution (liquid phase) or solid phase.

The functional groups of the constituent amino acids or amino acid mimetics must be protected during the coupling reactions to avoid undesired bonds being formed. The protecting groups that can be used are listed in Greene (1981) and "The Peptides: Analysis, Synthesis, Biology, Vol. 3, Academic Press, New York (1981), the disclosure of which is hereby incorporated by reference.

The alpha-carboxyl group of the C-terminal residue is usually protected by an ester that can be cleaved to give the carboxylic acid. These protecting groups include: 1) alkyl esters such as methyl and t-butyl, 2) aryl esters such as benzyl and substituted benzyl, or 3) esters which can be cleaved by mild base treatment or mild reductive means such as trichloroethyl and phenacyl esters. In the solid phase synthesis, the C-terminal amino acid is attached to an insoluble carrier (usually polystyrene). These insoluble carriers contain a group which will react with the carboxyl group to form a bond which is stable to the elongation conditions but readily cleaved later. Examples of which are oxime resin, chloro or bromomethyl resin, hydroxymethyl resin, and aminomethyl resin. Many of these resins are commercially available with the desired C-terminal amino acid already incorporated.

The alpha-amino group of each amino acid must be protected. Any protecting group known in the art can be used. Examples of these are: 1) acyl types such as formyl, trifluoroacetyl, phthalyl, and p-toluenesulfonyl; 2) aromatic carbamate types such as benzyloxycarbonyl (Cbz) and substituted benzyloxycarbonyls, 1-(p-biphenyl)-1-methylethoxycarbonyl, and 9-fluorenylmethyloxycarbonyl (Fmoc); 3) aliphatic carbamate types such as tert-butyloxycarbonyl (Boc), ethoxycarbonyl, diisopropylmethoxycarbonyl, and allyloxycarbonyl; 4) cyclic alkyl carbamate types such as cyclopentyloxycarbonyl and adamantyloxycarbonyl; 5) alkyl types such as triphenylmethyl and benzyl; 6) trialkylsilane such as trimethylsilane; and 7) thiol containing types such as phenylthiocarbonyl and dithiasuccinoyl. The preferred alpha-amino protecting group is either Boc or Fmoc. Boc is acid labile and Fmoc base labile protecting group. Many amino acid or amino acid mimetic derivatives suitably protected for peptide synthesis are commercially available.

The alpha-amino protecting group is cleaved prior to the coupling of the next amino acid. When the Boc group is used, the methods of choice are trifluoroacetic acid, neat or in dichloromethane, or HC1 in dioxane. The resulting ammonium salt is then neutralized either prior to the coupling or in situ with basic solutions such as aqueous buffers, or tertiary amines in dichloromethane or dimethylformamide. When the Fmoc group is used, the reagents of choice are piperidine or substituted piperidines in dimethylformamide, but any secondary amine or aqueous basic solutions can be used. The deprotection is carried out at a temperature between 0°C and room temperature.

Any of the amino acids or amino acid mimetics bearing side chain functionalities must be protected during the preparation of the peptide using any of the above-identified groups. Those skilled in the art will appreciate that the selection and use of appropriate protecting groups for these side chain functionalities will depend upon the amino acid or amino acid mimetic and presence of other protecting groups in the peptide, polypeptide or peptidomimetic. The selection of such a protecting group is important in that it must not be removed during the deprotection and coupling of the alpha-amino group.

For example, when Boc is chosen for the alpha-amine protection the following protecting groups are acceptable: p-toluenesulfonyl (tosyl) moieties and nitro for arginine; benzyloxycarbonyl, substituted benzyloxycarbonyls, tosyl or trifluoroacetyl for lysine; benzyl or alkyl esters such as cyclopentyl for glutamic and aspartic acids; benzyl ethers for serine and threonine; benzyl ethers, substituted benzyl ethers or 2-bromobenzyloxycarbonyl for tyrosine; p-methylbenzyl, p-methoxybenzyl, acetamidomethyl, benzyl, or t-butylsulfonyl for cysteine; and the indole of tryptophan can either be left unprotected or protected with a formyl group.

When Fmoc is chosen for the alpha-amine protection usually tert-butyl based protecting groups are acceptable. For instance, Boc can be used for lysine, tert-butyl ether for serine, threonine and tyrosine, and tert-butyl ester for glutamic and aspartic acids.

Once the elongation of the peptide, polypeptide or peptidomimetic, or the elongation and cyclization of a cyclic peptide or peptidomimetic is completed all of the protecting groups are removed. For the liquid phase synthesis the protecting groups are removed in whatever manner as dictated by the choice of protecting groups. These procedures are well known to those skilled in the art.

When a solid phase synthesis is used to synthesize a cyclic peptide or peptidomimetic, the peptide or peptidomimetic should be removed from the resin without simultaneously removing protecting groups from functional groups that might interfere with the cyclization process. Thus, if the peptide or peptidomimetic is to be cyclized in solution, the cleavage conditions need to be chosen such that a free a-carboxylate and a free a-amino group are generated without simultaneously removing other protecting groups. Alternatively, the peptide or peptidomimetic may be removed from the resin by hydrazinolysis, and then coupled by the azide method. Another very convenient method involves the synthesis of peptides or peptidomimetics on an oxime resin, followed by intramolecular nucleophilic displacement from the resin, which generates a cyclic peptide or peptidomimetic. When the oxime resin is employed, the Boc protection scheme is generally chosen. Then, the preferred method for removing side chain protecting groups generally involves treatment with anhydrous HF containing additives such as dimethyl sulfide, anisole, thioanisole, or p-cresol at 0° C. The cleavage of the peptide or peptidomimetic can also be accomplished by other acid reagents such as trifluoromethanesulfonic acid/trifluoroacetic acid mixtures.

Unusual amino acids used in this invention can be synthesized by standard methods familiar to those skilled in the art ("The Peptides: Analysis, Synthesis, Biology, Vol. 5, pp. 342-449, Academic Press, New York (1981)). N-Alkyl amino acids can be prepared using procedures described in previously (Cheung et al., 1977, Freidinger et al., 1982), which are incorporated herein by reference.

Additional synthetic procedures that can be used by one of skill in the art to synthesize the peptides, polypeptides and peptidomimetics targeting moieties are described in U.S. Pat. No. 5,879,657, the contents of which are herein incorporated by reference.

In this context it is appreciated that a variety of reagents may be suitable for use in the present methods, so long as these reagents posses the requisite biological activity. These reagents are generically referred to mimetics because they possess the ability to "mimic" a peptide domain involved in the functional interaction with the hK2 enzyme, and thereby interfere with (i.e., inhibit) normal function of hK2.

A hK2 peptide mimetic is any molecule, which exhibits the above-described properties. It can be a synthetic peptide, an analog or derivative of a peptide, a compound, which is shaped like the binding pocket of the above-described binding domain, such as an organic mimetic molecule, or other molecule.

The design of a hK2 peptide mimetic can be conducted by any of a variety of structural analysis methods for drug-design known in the art, including molecular modelling, two-dimensional nuclear magnetic resonance (2-D NMR) analysis, x-ray crystallography, random screening of peptide, peptide analog or other chemical polymer or compound libraries, and the like drug design methodologies.

In view of the broad structural evidence presented in the present specification, which shows that a hK2-inhibiting compound can be a polypeptide, a small polypeptide, a cyclic peptide, a derivative peptide, an organic peptidomimetic molecule, or a monoclonal antibody, that are diversely different chemical structures which share the functional property of selective inhibition of hK2 enzyme, the structure of a hK2-inhibiting compound useful in the present methods need not be so limited, but includes any organic hK2-inhibiting compound, as defined herein.

Preferably the organic hK2-inhibiting compound is an organic peptidomimetic compound having a basic group and an acidic group spaced from one another by a distance in the range of about 10 Angstroms to about 100 Angstroms.

Many of the compounds may include chiral centers and can exist in optically active, isomeric forms.

### Screening of Peptidomimetics

Peptides of the present invention may be used to screen for molecules that bind to hK2(tällä kai tarkoitetaan hK2 proteiinia, josta muualla käytetty vain nimitystä hK2 ilman proteiini- tai polypeptidi-sanaa) or for molecules to which hK2 binds. The binding of the molecule may inhibit (antagonist) or decrease the activity of the hK2. Examples of such molecules include small molecules.

Preferably, the molecule is closely related to the peptides of the present invention, e.g., a structural or functional mimetic. (See, Coligan et al., 1991). Similarly, the molecule can be closely related to the peptides of the present invention. In either case, the molecule can be rationally designed using known techniques.

The screening for these molecules can involve producing appropriate cells, which express the hK2. Preferred cells include cells from mammals, yeast, Drosophila, or E. coli. Cells expressing the hK2 are then contacted with a test compound potentially containing the molecule to observe binding or inhibition of activity of either the polypeptide or the molecule.

The assay may simply test binding of a candidate compound to the hK2 polypeptide, wherein the compound is labeled so that it can be detected, or the assay involves competition with a labeled competitor. Further, the assay may test whether the candidate compound results in a signal generated by binding to the polypeptide.

Preferably, the assay can be carried out using cell-free preparations, hK2 polypeptide/molecule affixed to a solid support, chemical libraries, or natural product mixtures. The assay may also simply comprise the steps of mixing a candidate compound with a solution containing the hK2, measuring hK2 activity or binding of the compound (as above).

Alternatively hK2-binding compounds can be attached to solid phase and binding of hK2 to those can be detected. HK2 can be labeled or it can be detected by antibody or other methods known by those skilled in the art.

Preferably, hK2 activity can be measured by using hK2 substrates, cleavage of which causes detectable change, like change in color or fluorescence.

An ELISA assay can measure hK2 activity in a sample (e.g., biological sample) using a monoclonal or polyclonal antibody. The antibody can measure hK2 level or activity by either binding, directly or indirectly, to the hK2 or by competing with the polypeptide for a substrate.

Moreover, the techniques of gene-shuffling, motif-shuffling, exon-shuffling, and/or codon-shuffling (collectively referred to as "DNA shuffling") may be employed to modulate the activities of the peptides of the present invention thereby effectively generating antagonists of the hK2 polypeptide. See generally, U.S. Pat. Nos. 5,605,793, 5,811,238, 5,830,721, 5,834,252, and 5,837,458; each of these patents are hereby incorporated by reference). In one embodiment, alteration of polynucleotides and corresponding polypeptides may be achieved by DNA shuffling. DNA shuffling involves the assembly of two or more DNA segments into a desired molecule by homologous, or site-specific, recombination. In another embodiment, polynucleotides and corresponding polypeptides may be altered by being subjected to random mutagenesis by error-prone PCR, random nucleotide insertion or other methods prior to recombination. In another embodiment, one or more components, motifs, sections, parts, domains, fragments, etc., of the peptides of the present invention may be recombined with one or more components, motifs, sections, parts, domains, fragments, etc. of one or more heterologous molecules.

The above scheme is principally applicable for some proteases, such as MMPs.

All these above assays can be used as diagnostic or prognostic markers. The molecules or peptidomimetics discovered using these assays can be used to treat disease or to bring about a particular result in a patient (e.g., inhibition of blood vessel growth) by inhibiting the hK2 polypeptide or enzyme activity.

Therefore, the invention includes a method of identifying compounds/peptidomimetics, which bind to the hK2 polypeptide comprising the steps of: (a) incubating a candidate binding compound with hK2 polypeptide; and (b) determining if binding has occurred. Moreover, the invention includes a method of identifying antagonists comprising the steps of: (a) incubating a candidate compound with the hK2a polypeptide, (b) assaying a biological activity, and (b) determining if a biological activity of the hK2 polypeptide has been altered.

The following non-limiting examples illustrate the invention further.

### Materials and Methods

### Materials

The monoclonal anti-PSA antibody 5E4 (Leinonen et al. 2002) and recombinant wild-type (wt) hK2 (Lovgren et al. 1999) were produced as described. PSA was purified from human seminal plasma as described (Zhang et al. 1995). M13 Bacteriophage Coat Proteins polyclonal antibody (ab6188) was purchased from Abcam (Cambridge, UK). The proteinases bovine trypsin and α-chymotrypsin were obtained from Athens Research and Technology, Athens, GA, USA. Human plasmin, thrombin and plasma kallikrein were from Sigma. Recombinant human trypsin was produced in-house. The chromogenic substrates S-2586, S-2222 S-2238, S-2403, S-2302, for chymotrypsin, trypsin, thrombin, plasmin and kallikrein, respectively, were from Chromogenix Instrumentation Laboratory (Milano, Italy).

### Phage display library screening

The phage display libraries were constructed in fUSE-5-phage as previously described (Koivunen et al. 1994). We screened libraries with the structures CX₇C, CX₈C, X₉, X₁₀, X₁₁, CX₃CX₃CX₃C, CX₈, CX₉, X₂CX₁₁C, where C stands for cysteine and X for any of the 20 randomly occurring amino acids. Screening was performed according to Koivunen *et al.* (Koivunen et al. 1994). We screened each peptide library against hK2, which was captured by MAb 5E4 coated into microtiter wells (Wu, Leinonen et al. 2000). MAb 5E4 binds PSA and hK2 in an equimolar manner and reacts with an epitope which is remote from the active site of these enzymes (Stenman et al. 1999), (Leinonen, Wu et al. 2002). Twenty four ng of hK2 was incubated in antibody-coated wells for 30 minutes at 37°C in Tris/NaCI buffer followed by washing of unbound hK2. An aliquot of phage libraries containing 10¹⁰-10¹¹ transducing units was added into the microtiter wells in 100 µL of 1% BSA in TBS, which was composed of 0.05 mol/L Tris buffer (pH 7.7) containing 0.154 mol/L NaCI and 8 mmol/L NaN3. Incubation took place at 4°C with gentle shaking for 16-24 hours in the first round and for 1 hour for the following three rounds. The phages were removed and wells were washed with TBS containing 0.5 % Tween 20. For elution of the bound phage 0.1 M Glycine-HCI, pH 2.2 containing 0.1% BSA was added. The eluted phage suspension was neutralized with 1 M Tris buffer, pH 9.0, amplified by infection of *Escherichia coli* K91 cells and purified by polyethylene glycol precipitation (Wu, Leinonen et al. 2000). After four rounds of selection and amplification single stranded DNA from individual phage clones was prepared. The peptide sequences were determined after amplification by PCR (Koivunen et al. 2001). The relevant part of the viral DNA was sequenced with an ABI 310 Genetic analyzer and Dye Terminator Cycle Sequencing Core Kit (PE Applied Biosystems, Foster City, CA, USA) using the primer 5'-TAA TAC GAC TCA CTA TAG GGC AAG CTG ATA AAC CGA TAC AAT-3' (SEQ ID NO: 130).

### Immunofluorometric assays (IFMAs)

The solid phase antibodies used in the IFMA were coated onto microtitration wells at a concentration of 5 mg/L in TBS for 16 h at 22°C, the solution was discarded and the wells were saturated with 10 g/L bovine serum albumin in TBS for 3 h at 22 °C. The antibodies used as tracers were labeled with a Eu³⁺ chelate as described (Hemmilä et al. 1984). The assay buffer was TBS, pH 7.7, containing 33 µmol/L bovine serum albumin and 1 µmol/L bovine gamma? globulin.

The phage IFMA was performed essentially as described (Wu, Leinonen et al. 2000). In this assay, 50 ng of hK2 in 200 µL of assay buffer was captured onto wells coated with MAb 5E4. After washing, 2.5 µL of phage ( 10⁸-10⁹ infectious particles) and 200 µL assay buffer were added to wells. After incubation for I h, the wells were washed and filled with 200 µL of assay buffer containing 100 ng Eu-labeled anti-phage polyclonal antibody recognizing the M 13 coat protein of the phage. After incubation for 60 min, the wells were washed 4 times, and enhancement solution (Perkin Elmer-Wallac) was added. The fluorescence was quantified with a Victor fluorometer in time resolved mode (Perkin Elmer-Wallac).

### Cross-inhibition test

The relative binding sites of the peptides on hK2 were mapped by competition experiments with chemically synthesized and phage peptides. The protocol was based on the IFMA method described above except that phages and the synthetic peptides were reacted together with hK2. Briefly, each of the synthetic peptides at 5 µM concentration was added into the wells in which hK2 had been captured by MAb 5E4. After incubation for 30 min each phage was added. After 1 h incubation, the wells were washed and the assay was as described above for IFMA.

For determination of hK2 immunoreactivity, an IFMA based on MAbs 5E4 and H50 developed by immunizing with PSA, but showing equimolar reactivity towards hK2 was used (Leinonen et al. 1999). In this IFMA, MAb 5E4 was used as a catcher and H50 as a tracer.

### Peptide synthesis

Peptides hK2p01 - 06 (Table 4) were synthesized with an Apex 396 DC multiple peptide synthesizer (Advanced ChemTech) with Fmoc strategy, TBTU/ DIPEA as the coupling reagent and Gly-Wang resin for all peptides (Advanced ChemTech). The side-chain protecting groups used in synthesis were; t-Butyloxycarbonyl (Boc) for Trp and Lys, tert-Butyl (tBu) for Ser and Thr. 2,2,4,6,7-Pentamethyl-dihydrobenzofurane-5-sulfonyl (Pbf) for Arg, trityl (Trt) for GIn. For Cys both Acetamidomethyl (Acm) and Trt proctetion groups were used.

The structure was verified by MALDI-TOF mass spectrometry (Bruker, Germany) and the purity was determined by analytical HPLC with a 240 x 1.4 mm C₁₈ column eluted with 0 - 60% ACN in 0.1 % TFA for 30 minutes.

The alanine replacement sets were done for peptides hK-2p01 - p03 by replacing other amino acids for alanine one by one.

**Cyclization of the peptides:** For cyclization, two additional cysteins, viz. carboxy terminal cystein with a conventional trityl (Trt) side chain protection group and an amino terminal with an Acetaminomethyl (Acm) side chain protection group, were incorporated into a number of peptides according to the invention. The amino acid sequences of these modified peptides are indicated in Table 5. The peptides were cyclised by using the iodination method. Thus, a lyophilized peptide was dissolved in 50% acetic acid (AcOH) at a concentration of 2 mg / ml. I M HC1 (0.1 ml/mg of peptide) was added, followed immediately by 0.1 M iodine solution in 50% AcOH (5 eq. / Acm). The solution was stirred vigorously at room temperature for 40 minutes. The reaction was terminated with 0.1 M sodium thiosulphate. After filtering on a filter having an average pore size of 0.45 µm, the peptides were purified with HPLC as described above. The formation of disulphide bonds was verified by MALDI-TOF mass spectrometry (Bruker analytic GMBH, Karlsruhe, Germany).

The inhibitory activity of the peptides was tested as described above.

### Effect of the peptides on the enzyme activity of hK2

The effect of the peptides on the enzyme activity of hK2 was studied by using the chromogenic substrate S-2302 (H-D-Pro-Phe-Arg-pNA). HK2 (0.17 µM) was incubated with a 100-1000-fold molar excess of synthetic peptides in 20 mM Tris-buffer pH 8.0 containing 0.1 % BSA for 30 minutes at room temperature. After addition of the substrate to a final concentration of 0.2 mM the absorbance was monitored at 405 nm at 5-10 min intervals for 1 h on a Victor 1420 Multilabel fluorometer (PerkinElmer-Wallac, Turku, Finland)

The inhibition of hK2 by the peptides was compared by determining IC50 constants for hK2 inhibition, which were obtained by determining the velocity of the cleavage of S-2302 substrate at 200 µM concentration by 0.17 µM hK2 *at 0- 400 µM peptide* concentrations. In addition, Ki value for the most potent inhibitory peptide were determined from Lineweaver-Burke plots after determining the velocity of the cleavage *of 0-400 µM* S-2302 substrate by 0.2 µM hK2 *at 4 µM peptide* concentration.

The specificity of the peptides was analyzed by studying their effect on the activity of other serine proteases including α-chymotrypsin, bovine trypsin, human trypsin 1, trypsin 2 and trypsin 3, urokinase, thrombin, plasmin, plasma kallikrein and PSA. The assay was as described above for hK2 except that the substrate for PSA and α-chymotrypsin was S-2586 (MeO-Suc-Arg-Pro-Tyr-pNA)(Chromogenix), S-2444 for urokinase, S-2238 for thrombin, S-2403 for plasmin, S-2302 for plasma kallikrein and that for various forms of trypsin was S-2222 (CO-Ile-Glu-(OR)-Gly-Arg-pNA)(Chromogenix).

### Similarity searches

BLAST searches were performed to find if the peptides isolated had similarity to the structures of previously identified peptides or proteins.

### Development of DTPA and USPIO (ultra small particles of iron oxide) conjugates from hK2 peptides

Primary amino derivatized 50 nm USPIO particles (Kisker GbR, Steinfurt) are labeled with hK2 and PSA specific peptides via additional cystein in the amino terminus by using bifunctional coupling reagent MBS (maleimimidodihydroxysuccinimide <-tämä oikein, kummatkin kyllä käy ester vai m-Maleimidobenzoyl-N-hydroxysuccinimide ester???). Peptides are labeled with isothiocyanate derivatzed DTPA molecules via amino terminal primary amine or carboxy terminal lysine on resin. The side chain is protected with Mtt-protection group (5-methoxytrityl), which can be removed with 1% TFA in DCM without removing whole peptide from the resin. After cleavage from resin DTPA-peptide conjugates are labeled with Technetium or with Gadolinium (Qu et al. 2001). Different chelators and different peptides together influence the *in vitro* and mouse *in vivo* properties of ⁹⁹Tc^{m}. Nucl.Med. Comm. 2001, 22:203-215.

The biodistribution and the ability to localize tumor tissue will be tested by using xenografted prostate cancer tumor in mice and localization will be monitored the MRI (magnetic resonance imaging) and SPECT (Single positron emission computerized tomography). A set of the variants from hK2 peptide will be synthesized in order to study the stability of the molecules in vivo in order to develop optimal structures for therapeutic purposes.

### Plasma resistance test

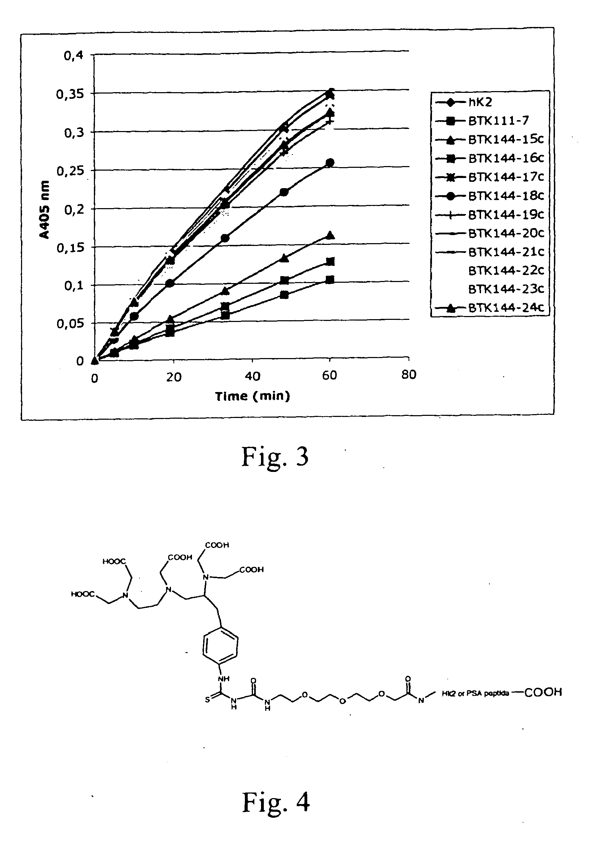

In order to further study the plasma resistance, a set of linear and cyclic peptides are synthezised. In these peptides certain amino acid positions are replaced with D-amino acids or unnatural components. Peptides are synthesized by using conventional solid phase synthesis method with Fmoc strategy. DTPA-ITC (isothiocyanate) is coupled to the free amino terminus of the peptide. HK2 specific peptides are cyclized with sulphur bridge by using asetamidomethyl (Acm) protecting group and the iodine method (Figure 4).

Both original peptides and variants will be tested with mouse and human sera. Briefly, peptides are incubated with 20% - 33% serum dilution for the indicated times. After stopping the reaction with Trifluoroacetic acid, samples are analyzed by mass spectrometry. Hinke et al.(2004).

### Xenografted mice

The human prostate cancer cell line LNCaP is used in this study. One million cells are suspend in 100 µl of PBS and further mixed with 0.30 mL Matrigel (BD Biosciences-Labware, Bedford, MA, USA). The cell suspension is injected subcutaneously in nude mice and the tumors are growing for 2 to 6 weeks.

### MRI

One mg of USPIO conjugates or DTPA(Gd3+) conjugates in 1 ml PBS are added on site or i.v. and are incubated for 1 to 6 hours. Animals are sacrificed and perfused. Animals are monitored by using MRI, T1 weighted scanning for Gadolinium and T2 weighted scanning for USPIO.

### SPECT

About 100 megaBecquerel in 500 µl of PBS is injected intravenously or subcutaneously. and after 1 to 6 hours the animals are sacrificed and perfused before SPECT/PET imaging.

### Results

### Isolation and structure of the peptides

When screening with 10 different phage display peptide libraries was performed, phages reacting with hK2 were obtained from libraries with the structures X10 and X11. Peptide sequence for 24 and 10 clones from X10 and X11 libraries, respectively, are shown in Table 4. Four different hK2-binding peptides were identified from the X10-library, designated HK2p01 - HK2p04, of which the most prevalent one was HK2p02 (AARRPFPAPS; SEQ ID NO:2). Three of these peptides were linear (HK2p01 - HK2p03). HK2p04 contained two Cys residues indicating that it forms a cyclic structure through a disulfide bridge. Two peptide sequences (FRTCLRSWACM; SEQ ID NO:5, and CYRMPTCMQRD; SEQ ID NO:6) were isolated from the X11 library and they contained two Cys residues indicating that they also are cyclic.

**Table 4. Amino acid sequences of hK2-binding peptides**

| The purified single stranded DNA of the clones were sequenced and identified from phage display libraries X10 and X11 after four successive rounds. | | | | |
|---|---|---|---|---|
| Code | Display Library | Peptide Sequence | No. Isolates | SEQ ID NO |
| HK2p01 | X10 | SRFKVWWAAG | 1 | 1 |
| HK2p02 | X10 | AARRPFPAPS | 19 | 2 |
| HK2p03 | X10 | PARRPFPVTA | 1 | 3 |
| HK2p04 | X10 | CFRQGCWVIT | 3 | 4 |
| HK2p05 | X11 | FRTCLRSWACM | 6 | 5 |
| HK2p06 | X11 | CYRMPTCMQRD | 4 | 6 |

Common motifs can be found among the peptides, i.e., peptides HK2p02 and HK2p03 both contain the motif ARRPFP (SEQ ID NO:95) and when the three cyclic peptides HK2p04, HK2p05 and HK2p06 are aligned according to the location of the disulfide bridges, they all have an Arg-residue as the third residue after the first Cys-residue. Furthermore, in peptides HK2p05 and HK2p06 the second Cys residue is followed by a Met residue. Arg is present in all peptides and this residue is known to be located in the P1-position of hK2-substrates (Cloutier et al. 2002).

Table 5 gives the amino acid sequences of the cyclic peptides prepared as described above.

**Table 5. Amino acid sequences of the control and the cyclic hK2 specific peptide inhibitors. C3 is Cystein with an Acm protection group.**

| Code | Sequence | MW | Type | SEQ ID NO |
|---|---|---|---|---|
| | | | hK2 (b) | 75 |
| BTK111-7 | ARFKVWWG | 1048, 56 | lin ??? | |
| BTK144-16 | GC3AARFKVWWAACG | 1525,806 | hK2(b) | 84 |
| BTK144-17 | C3ARRPAPAPCG | 1098,303 | hK2 (a) | 85 |
| BTK144-18 | C3AARRPAPAPCG | 1169,382 | hK2 (a) | 86 |
| BTK149-19 | C3AAARRPAPAFCG | 1240,461 | hK2 (a) | 87 |
| BTK144-20 | C3RRPAPACG | 930,108 | hK2 (a) | 88 |
| BTK144-21 | C3ARRPAPACG | 1001,187 | hK2 (a) | 89 |
| BTK144-22 | C3ARRPAPAACG | 1072,265 | hK2(a) | 90 |
| BTK144-23 | C3AARRPAPAACG | 1143,344 | hK2 (a) | 91 |
| BTK144-24 | C3AAARRPAPAACG | 1214, 423 | hK2 (a) | 92 |

### Inhibition of phage binding with synthetic peptides

The relative binding specificity of the five peptides was studied by inhibition experiments using phage and corresponding synthetic peptides. Synthetic peptides HK2p01, 02, 03 and 06 inhibited efficiently the binding of the phage carrying the corresponding peptide as part of pIII protein showing that the peptides were active without the protein fusion part (Fig 1). Phage p05 showed weak binding in phage IFMA and phage binding was not inhibited by the corresponding synthetic peptide. Thus, this peptide was not further studied. In cross-inhibition experiments synthetic peptides p01 - p03 inhibited efficiently the binding of all five phages studied indicating that they bind to the same place or close to each other on hK2 (Fig 1).

### Effect of the peptides on enzyme activity

Synthetic peptides HK2p01-HK2p06 were studied for their effect on enzyme activity of hK2, PSA, chymotrypsin, bovine trypsin, human trypsin 1-3, plasmin, thrombin, urokinase and plasma kallikrein. Incubation of hK2 with the peptides HK2p01, HK2p02 and HK2p03 caused a significant reduction of the enzyme activity (Fig 2). When using 0.17 µM hK2, a slight inhibition was observed with 100-fold molar excess of peptide and with a 1000-fold excess inhibition was 70 - 80%. Peptides HK2p04 and HK2p06 did not show any effect on the enzyme activity of hK2, hence they are not shown in the figures. Peptides HK2p01, HK2p02 and HK2p03 showed no inhibition on the other serine proteinases studied, i.e., PSA, α-chymotrypsin, bovine trypsin, human trypsin 1-3, plasmin, thrombin, urokinase and plasma kallikrein. Fractionation of the reaction mixture containing trypsin and the hK2-binding peptide by C18 chromatography showed that the peptides remained intact in the presence of bovine trypsin (data not shown).

### Amino acid substitution and deletion analysis

To define the motifs required for inhibitory activity of the peptides amino acid substitution and deletion analysis was performed for peptides p01-p03. In the deletion analysis, each peptide was shortened either from N- or from C-terminus one amino acid at a time. The results are shown in Table 6.

In peptide p01 (SRFKVWWAAG; SEQ ID NO:1) Ala-replacement of R2, F3, K4, V5, W6 or W7 individually strongly reduced the ability of the peptide to inhibit hK2 suggesting that these residues are key mediators of the activity. Replacement of S 1 with A increased the inhibitory activity of the peptide significantly, while deletion of this Ser led to partial loss of activity. Thus, some mutations in the amino terminal amino acid are tolerated; it is essential that a spacer amino acid is included before R2. Deletion of the amino terminal SR-motif abolished the activity confirming the essential role of the R2-residue. Deletion analysis further showed that the AAG-motif in the C-terminus of p01 is not required for inhibition of hK2 (Table 6). The IC50 of the peptide decreased from 3.4 µM to 2.2 µM, i.e. there was a clear increase in the inhibitory potency of the mutated peptide as compared to that of the non-mutated peptide. Thus, substitution and deletion analyses indicate that the RFKXW motif (SEQ ID NO:96) is required for inhibition of hK2, but maximal inhibition is achieved with the ARFKVWWAAG motif (SEQ ID NO:56). This was confirmed by showing that with the peptide ARFKVWWAAG the lowest IC50 (2.2 µM) was observed. The results of the substitution and deletion analyses are summarized in Table 6.

The effect of alanine substitutions on the inhibitory activity of peptides p02 (AARRPFPAPS) and p03 (PARRPFPVTA) is shown in Table 6. In both peptides, substitution of R3, R4, P5 and P7 strongly reduced the inhibitory activity. Substitution of P1 or V8 in p03 slightly increased the activity (Table 6). Deletion of P from amino terminus of p02 led to partial reduction of peptide activity, while deletion of PA abolished activity. The APS and VTA-motifs could be deleted from the C-terminus of p02 and p03, respectively, without significant reduction of activity. These results suggest that the ARRPXP motif (SEQ ID NO:94), which is present in both p02 and p03, represents a particularly preferred embodiment for hK2 inhibition. The IC50 values determined for peptides p01 - p03 and some of their derivatives are shown in Table 6. The strongest inhibitor for hK2 is peptide ARFKVWWAAG with an IC50 value of 2.2 µM and a Ki value of 1.41 µM.

**Table 6. Percent inhibition of hK2 binding peptide derivatives based on alanine-scanning and sequence trimming. The sequences are shown in one-letter code of the amino acids with their molecular weights and their hK2-inhibitory effect presented in percentage inhibition. The effect of these derivatives was observed by measuring the enzymatic activity of hK2 using chromogenic substrate at a wavelength of 405 nm. For clarity, the amino acid residues have been separated by commas in the peptide sequences.**

| **Sequence** | **MW** | **%** **inhibition** | **SEQ ID** **NO** |
|---|---|---|---|
| G, A, A, R, R, P, F, P, A, P, S, G | 1183.34 | 77% | 7 |
| G, A, A, A, R, P, F, P, A, P, S, G | 1098.23 | 0% | 8 |
| G, A, A, R, A, P, F, P, A, P, S, G | 1098.23 | 0% | 9 |
| G, A, A, R, R, A, F, P, A, P, S, G | 1157.30 | 0% | 10 |
| G, A, A, R, R, P, A, P, A, P, S, G | 1107.24 | 75% | 11 |
| G, A, A, R, R, P, F, A, A, P, S, G | 1157.30 | 24% | 12 |
| G, A, A, R, R, P, F, P, A, A, S, G | 1157.30 | 69% | 13 |
| G, A, A, R, R, P, F, P, A, P, A, G | 1167.34 | 78% | 14 |
| | | | |
| G, P, A, R, R, P, F, P, V, T, A, G | 1225.42 | 49% | 15 |
| G, A, A, R, R, P, F, P, V, T, A, G | 1199.38 | 61% | 16 |
| G, P, A, A, R, P, F, P, V, T, A, G | 1140.31 | 0% | 17 |
| G, P, A, R, A, P, F, P, V, T, A, G | 1140.31 | 0% | 18 |
| G, P, A, R, R, A, F, P, V, T, A, G | 1199.38 | 0% | 19 |
| G, P, A, R, R, P, A, P, V, T, A, G | 1149.32 | 49% | 20 |
| G, P, A, R, R, P, F, A, V, T, A, G | 1199.38 | 17% | 21 |
| G, P, A, R, R, P, F, P, A, T, A, G | 1197.36 | 58% | 22 |
| G, P, A, R, R, P, F, P, V, A, A, G | 1195. 39 | 45% | 23 |
| | | | |
| G, P, A, R, R, P, F, P, V, T, G | 1154.28 | 48% | 24 |
| G, P, A, R, R, P, F, P, V, G | 1053.17 | 37% | 25 |
| G, P, A, R, R, P, F, P, G | 954.04 | 33% | 26 |
| G, P, A, R, R, P, F, G | 856.92 | 0% | 27 |
| G, P, A, R, R, P, G | 709.74 | 4% | 28 |
| G, P, A, R, R, G | 612.62 | 8% | 29 |
| G, P, A, R, G | 456.43 | 0% | 30 |
| | | | |
| G, P, A, R, R, P, F, P, V, T, A, G | 1225.36 | 58% | 31 |
| G, A, R, R, P, F, P, V, T, A, G | 1128.24 | 27% | 32 |
| G, R, R, P, F, P, V, T, A, G | 1057.16 | 2% | 33 |
| G, R, P, F, P, V, T, A, G | 900.97 | 0% | 34 |
| G, P, F, P, V, T, A, G | 744.78 | 0% | 35 |
| G, F, P, V, T, A, G | 647.66 | 0% | 36 |
| G, P, V, T, A, G | 500.48 | 0% | 37 |
| G, V, T, A, G | 403.36 | 0% | 38 |
| R, R, P, A, P | 595.70 | 0% | 39 |
| R, R, P, F, P | 671.80 | 0% | 40 |
| R, R, P, A, G | 555.60 | 0% | 41 |
| R, R, P, F, G | 631.70 | 0% | 42 |
| G, R, R, P, A, P, G | 709.74 | 0% | 43 |
| G, R, R, P, F, P, G | 785.84 | 0% | 44 |
| A,A, R, R, P, A, P, G | 794.88 | 36% | 45 |
| A, A, A, R,R, P, A, P, G | 865.96 | 51% | 46 |
| A, A, R, R, P, F, P, G | 870.98 | 28% | 47 |
| A, A, A, R, R, P, F, P, G | 942.06 | 37% | 48 |
| Ac, A, A, A, R, R, P, A, P, A | 922.02 | 43% | 49 |
| Ac, A, A, A, R, R, P, A, P, G | 907.96 | 49% | 50 |
| G, A, A, R, R, P, F, P, A, P, G | 1096.20 | 69% | 51 |
| G, A, A, R, R, Sar, F, P, A, P, G | 1070.08 | 0% | 52 |
| G, A, A, R, R, P, F, Sar, A, P, G | 1070.08 | 24% | 53 |
| G, A, A, R, R, Sar, F, Sar, A, P, G | 1043.96 | 0% | 54 |
| S, R ,F, K, V, W, W, A, A, G | 1207.35 | 79% | 55 |
| A, R , F, K, V, W, W, A, A, G | 1191.35 | 94% | 56 |
| S, A , F, K, V, W, W, A, A, G | 1122.24 | 27% | 57 |
| S, R ,A, K, V, W, W, A, A, G | 1131.25 | 0% | 58 |
| S, R , F, A, V, W, W, A, A, G | 1150.26 | 0% | 59 |
| S, R ,F, K, A, W, W, A, A, G | 1179.30 | 39% | 60 |
| S, R ,F, K, V, A, W, A, A, G | 1092.22 | 10% | 61 |
| S, R , F, K, V, W, A, A, A, Gv | 1092.22 | 30% | 62 |
| S, R ,F, K, V, W, W, A, A, G | 1207.35 | 78% | 63 |
| S, R ,F, K, V, W, W, A, A, Gv | 1207.35 | 85% | 64 |
| R ,F, K, V, W, W, A, A, G | 1120.27 | 22% | 65 |
| F, K, V, W, W, A, A, G | 964.08 | 0% | 66 |
| W, W, A, A, G | 589. 60 | 0% | 67 |
| S, R , F, K, V, W, W, A, G | 1136.27 | 80% | 68 |
| S, R , F, K, V, W, W, G | 1065.19 | 79% | 69 |
| S, R ,F, K, V, W, G | 878.98 | 8% | 70 |
| S, R ,F, K, V, G | 692.77 | 7% | 71 |
| S, R , F, K, G | 593.64 | 0% | 72 |
| S, R , F, K, V, W, W, G | 1065.19 | 82% | 73 |
| Ac, S, R ,F, K, V, W, W, G | 1107.19 | 86% | 74 |
| A, R , F, K, V, W, W, G | 1049.19 | 94% | 75 |
| Ac, A, R , F, K, V, W, W, G | 1091.19 | 93% | 76 |
| Ac, A, R F, K, V, W, W, G, G | 1148.21 | 92% | 77 |
| A, R F, K, V, W, W, G, G | 1106.21 | 93% | 78 |
| A, R F, K, V, W, W, A | 1063.25 | 95% | 79 |
| Ac, A, R , F, K, V, W, W, A | 1105.25 | 94% | 80 |
| Ac, A, R , F, K, V, W, W, A, A | 1176.33 | 93% | 81 |
| A, R F, K, V, W, W, A, CONH₂ | 1062.25 | 86% | 82 |

In order to study the effect of the length of the peptides on the biological activity of the hK2 type, a series of peptides with additional alanines in both ends of the peptide was synthesized (Table 5).

Some of the cyclic peptides were inactive, but BTK144-18c with two alanines showed slight inhibitory effect (Figure 3). Furthermore, two variants of the cyclic hK2 type b (mikä on type b?) peptides inhibited clearly hK2 protease activity when compared to the linear control peptide BTK111-7 with known inhibitory activity (Table 5/6).

### References

Anwer et al. (1990), Int. J Pep. Protein Res. 36:392-399
Arap, W, Pasqualini R, Ruoslahti E. (1998) Cancer treatment by targeted drug delivery to tumor vasculature in a mouse model [see comments]. Science 279:377-80.
Bodanszky (1988). Peptide Chemistry: A Practical Textbook, Springer-Verlag, New York
Bodanszky et al. (1984). The Practice of Peptide Synthesis, Springer-Verlag, New York
Boerman, O. C., W. J. Oyen, et al. (2000). "Radio-labeled receptor-binding peptides: a new class of radiopharmaceuticals." Semin Nucl Med 30(3): 195-208.
Chapdelaine, P., G. Paradis, et al. (1988). "High level of expression in the prostate of a human glandular kallikrein mRNA related to prostate-specific antigen." FEBS Lett 236(1): 205-8.
Cheung et al., (1977) Can. J. Chem. 55, 906.
Chodak, G. W., R. A. Thisted, et al. (1994). "Results of conservative management of clinically localized prostate cancer [see comments]." N Engl J Med 330(4): 242-8.
Cloutier, S. M., J. R. Chagas, et al. (2002). "Substrate specificity of human kallikrein 2 (hK2) as determined by phage display technology." Eur. J. Biochem. 269(1 1): 2747-5402.
Cloutier, S. M., C. Kundig, et al. (2004). "Development of recombinant inhibitors specific to human kallikrein 2 using phage-display selected substrates." Eur J Biochem 271(3): 607-13.
Coligan et al. (1991). Current Protocols in Immunology 1(2); Chapter 5.
Darson, M. F., A. Pacelli, et al. (1997). "Human glandular kallikrein 2 (hK2) expression in prostatic intraepithelial neoplasia and adenocarcinoma: a novel prostate cancer marker." Urology 49(6): 857-62.
Denis, L. and K. Griffiths (2000). "Endocrine treatment in prostate cancer." Semin Surg Oncol 18(1): 52-74.
Denmeade, S. R., A. Nagy, et al. (1998). "Enzymatic activation of a doxorubicin-peptide prodrug by prostate- specific antigen." Cancer Res 58(12): 2537-40.
Fechheimer et al. (1987). Proc. Natl. Acad. Sci. USA, 84:8463-8467.
Felgner (1997). Sci Am. 276(6):102-6.
Frenette, G., R. Tremblay, et al. (1997). "Prostatic kallikrein hK2, but not prostate-specific antigen (hK3), activates single-chain urokinase-type plasminogen activator." Int J Cancer 71(5): 897-9.
Freidinger et al., (1982) J. Org. Chem. 48, 77.
Gopal (1985). Mol. Cell Biol., 5:1188-1190.
Gross, Meienhofer, Udenfriend, Eds. (1980-1987) The Peptides: Analysis, Synthesis, Biology, Vol. 1, 2, 3, 5, and 9, Academic Press, New York
Greenn (1981), Protective Groups in Organic Synthesis, John Wiley & Sons, New York
Harland and Weintraub (1985). J. Cell Biol., 101:1094-1099.
Hemmilä, I., S. Dakubu, et al. (1984). "Europium as a label in time-resolved immunofluorometric assays." Anal. Biochem. 137: 335-343.
Hinke et al. (2004). JBC, 6(6): 3998-4006.
Houghten, RA, Pinilla C, Appel JR, Blondelle SE, Dooley CT, Eichler J, Nefzi A, Ostresh JM. Mixture-based synthetic combinatorial libraries. J Med Chem 1999; 42:3743-78.
Koivunen, E., W. Arap, et al. (1999). "Identification of receptor ligands with phage display peptide libraries." J Nucl Med 40(5): 883-8.
Koivunen, E., W. Arap, et al. (1999). "Tumor targeting with a selective gelatinase inhibitor." Nat Biotechnol 17(8): 768-74.
Koivunen, E., T.-M. Ranta, et al. (2001). "Inhibition of {beta}2 Integrin-mediated Leukocyte Cell Adhesion by Leucine-Leucine-Glycine Motif-containing Peptides." J. Cell Biol. 153(5): 905-916.
Koivunen, E., B. Wang, et al. (1994). "Peptides in cell adhesion research." Methods Enzymol 245: 346-69.
Koivunen, E., B. Wang, et al. (1994). "Isolation of a highly specific ligand for the alpha 5 beta 1 integrin from a phage display library." J. Cell Biol. 124(3): 373-380.
Lehninger, Biochemistry, Second Edition; Worth Publishers, Inc. New York:N.Y., 1975, pp.71-77]
Leinonen, J., M. Leinimaa, et al. (1999). "Reactivity of anti-PSA monoclonal antibodies with recombinant human kallikrein-2." Tumour Biol 20 Suppl 1: 35-7.
Leinonen, J., P. Wu, et al. (2002). "Epitope Mapping of Antibodies against Prostate-specific Antigen with Use of Peptide Libraries." Clin Chem 48(12): 2208-2216.
Liu, S. and D. S. Edwards (1999). "99mTc-Labeled Small Peptides as Diagnostic Radiopharmaceuticals." Chem Rev 99(9): 2235-68.
Lovgren, J., S. Tian, et al. (1999). "Production and activation of recombinant hK2 with propeptide mutations resulting in high expression levels." Eur. J. Biochem. 266(3): 1050-5402.
Merrifield (1963), J. Am. Chem. Soc., 85, 2149-2154.
Mikolajczyk, S., L. Millar, et al. (1999). "Prostatic human kallikrein 2 inactivates and complexes with plasminogen activator inhibitor-I." Int J Cancer 81(3): 438-42.
Morpurgo, M., C. Monfardini, et al. (2002). "Selective Alkylation and Acylation of alpha and epsilon Amino Groups with PEG in a Somatostatin Analogue: Tailored Chemistry for Optimized Bioconjugates." Bioconjug Chem 13(6): 1238-43.
Morris, B. J. (1989). "hGK-1: a kallikrein gene expressed in human prostate." Clin Exp Pharmacol Physiol 16(4): 345-51.
Nachman et al. (1995), Regul. Pept. 57:359-370
Nargund, RP, Patchett AA, Bach MA, Murphy MG, Smith RG (1998), Peptidomimetic growth hormone scretagogues. Design Considerations and therapeutic potential. J Med Chem; 41:3103-27
Nicoiau et al. (1987). Methods Enzymol., 149:157-176 Nucl. Med. Comm. 2001, 22:203-215
Pasqualini, R., E. Koivunen, et al. (1997). "Alpha v integrins as receptors for tumor targeting by circulating ligands." Nat Biotechnol 15(6): 542-6.
Qu T. Wang Y. Zhu Z. Rusckowsi M. Hnatowich D.J. Different chelators and different peptides together influence the in vitro and mouse in vivo properties of 99Tcm. Nucl. Med. Com 22:203-215, 2001.
Rippe et al. (1990). Mol. Cell Biol., 10:689-695.
Rivera-Baeza et al. (1996), Neuropeptides 30:327-333.
Roberts and Vellaccio (1983) The Peptides, 5: 342-429
Ruoslahti, E. and D. Rajotte (2000). "An Address System in the Vasculature of Normal Tissues and Tumors." Annu. Rev. Immunol. 18(1): 813-827.
Slepushkin, VA, Salem II, Andreev SM, Dazin P, Duzgunes N. Targeting of liposomes to HIV-1-infected cells by peptides derived from the CD4 receptor. Biochem Biophys Res Commun 1996; 227:827-33.
Stege, R., M. Grande, et al. (2000). "Prognostic significance of tissue prostate-specific antigen in endocrine-treated prostate carcinomas." Clin Cancer Res 6(1): 160-5.
Stenman, U. H., E. Paus, et al. (1999). "Summary report of the TD-3 workshop: characterization of 83 antibodies against prostate-specific antigen." Tumour Biol 20(Suppl 1): 1-12.
Stewart and Young (1984).Solid Phase Peptide Synthesis, 2nd ed, Pierce Chemical Co., Rockford, III.
Tur-Kaspa et al. (1986). Mol. Cell Biol., 6:716-718:
Yang et al. (1990). Proc. Natl. Acad. Sci USA, 87:9568-9572.
Wang, M. C., L. A. Valenzuela, et al. (1979). Purification of a human prostate specific antigen. Invest. Urol. 17: 159-163.
Wu, P., J. Leinonen, et al. (2000). "Identification of novel prostate-specific antigen-binding peptides modulating its enzyme activity." Eur. J. Biochem. 267(20): 6212-6220.
Wu, P., U. H. Stenman, et al. (2004). "Separation of enzymatically active and inactive prostate-specific antigen (PSA) by peptide affinity chromatography." Prostate 58(4): 345-53.
Wu, P., L. Zhu, et al. (2004). "Immunopeptidometric assay for enzymatically active prostate-specific antigen." Clin Chem 50(1): 125-9.
Wu and Wu (1993). Adv. Drug Delivery Rev., 12:159-167.
Zalipsky, S, Puntambekar B, Boulikas P, Engbers CM, Woodle MC (1998). Peptide attachment to extremities of liposomal surface grafted PEG chains: preparation for integrin-mediated gene delivery [see comments]. Hum Gene Ther 9:1037-47.
Zhang, W. M., J. Leinonen, et al. (1995). "Purification and characterization of different molecular forms of prostate-specific antigen in human seminal fluid." Clin Chem 41(11): 1567-73.

## Claims

1. An isolated peptide comprising
a) an amino acid sequence selected from the group consisting of: SRFKVWWAAG (SEQ ID NO:55), ARFKVWWAAG (SEQ ID NO:56), SAFKVWWAAG (SEQ ID NO:57), SRFKAWWAAG (SEQ ID NO:60), SRFKVAWAAG (SEQ ID NO:61), SRFKVWAAAG (SEQ ID NO:62), SRFKVWWAAG (SEQ ID NO:63), SRFKVWWAAG (SEQ ID NO:64), RFKVWWAAG (SEQ ID NO:65), SRFKVWWAG (SEQ ID NO:68), SRFKVWWG (SEQ ID NO:69), or
b) an amino acid sequence selected from the group consisting of: SRFKVWWG (SEQ ID NO:73), SRFKVWWG (SEQ ID NO:74), ARFKVWWG (SEQ ID NO:75), ARFKVWWG (SEQ ID NO:76), ARFKVWWGG (SEQ ID NO:77), ARFKVWWGG (SEQ ID NO:78), ARFKVWWA (SEQ ID NO:79), ARFKVWWA (SEQ ID NO:80), ARFKVWWAA (SEQ ID NO:81) and ARFKVWWA(CONH₂) (SEQ ID NO:82), or
c) an amino acid sequence, which is SRFKVWWAAG (SEQ ID NO: 1.

2. The peptide according to claim 1, further comprising carboxy terminal glycine or CONH₂, and/or amino terminal acetyl moiety.

3. The isolated peptide according to claim 1 or 2, further comprising a label, and/or a cytoxic agent attached to the peptide, said cytotoxic agent optionally comprising and anti-neoplastic pro-drug.

4. The isolated peptide according to any of claims 1 to 3, further comprising an imaging agent.

5. An isolated nucleic acid encoding the peptide according to any of claims 1 to 4

6. A method for inhibiting prostate tumour growth and/or spread, comprising a step of contacting in vitro the prostate cell with a nucleic acid comprising a nucleotide sequence of claim 5, encoding the peptide of any of claims 1 to 4, and further comprising a promoter active in said cell, wherein said promoter is operably linked to the nucleotide sequence encoding said peptide, under conditions permitting the uptake of said nucleic acid and expression of said peptide by said cell in an amount effective to inhibit growth and/or spread of said cell.

7. The method according to claim 6, wherein said nucleic acid is encapsulated in a liposome, wherein said nucleic acid preferably is a viral vector selected from the group consisting of retrovirus, adenovirus, adeno-associated virus, vaccinia virus and herpes virus.

8. A method of screening a biological sample for hK2, comprising the steps of:
a) contacting a biological sample suspected of containing hK2 protein with a composition comprising a peptide according to any one of claims 1 - 4; and
b) determining the binding and/or inhibition of proteolytic activity of said peptide with hK2.

9. The method according to claim 8, wherein the peptide comprises a detectable label, and the determining step comprises detecting the presence of the label bound to the biological sample.

10. The method according to claim 8 or 9, wherein the biological sample comprises mammalian tissue, and the determining step comprising determining the presence or quantity of peptide in tissue.

11. A method of imaging tissues that contain hK2 comprising:
a) contacting the tissue with a composition comprising a peptide of any of claims 1 - 4; and
b) imaging (cells that express) hK2 in said tissue by detecting said peptide bound to tissue.

12. The method according to claim 11, wherein said peptide comprises a detectable label, and wherein the imaging step comprises detecting the label in the tissue.

13. The method according to claim 11 or 12, wherein said tissue is human tissue, preferably a neoplastic tissue, particularly a prostate cancer.

14. A method of designing peptidomimetic compounds, comprising using a peptide according to any of claims 1 - 4 as a lead compound.

15. A method of deriving a peptidomimetic of a biologically active hK2 peptide comprising the steps of:
- selecting a biologically active hK2 peptide from any of the peptides according to claims 1 - 4, the hK2 peptide comprising at least a peptide sequence, wherein biological activity is related to at least two elements of such peptide, the at least two elements independently comprising an amino acid residue, amino acid side chain moiety or derivative thereof, and wherein the hK2 peptide is complexed to or associated to hK2 enzyme, whereby the hK2 peptide inhibits hK2 enzyme activity; and
- modeling a non-peptidic structure that is superimposable on the template space defined the hK2 peptide, and
- forming a peptidomimetic by adding to the non-peptidic structure at least one element comprising an amino acid residue, amino acid side chain moiety or derivative thereof, such at least an element occupies a similar descriptor space as corresponding element of the biologically active hK2 peptide.
